# FASCICULE DE BREVET EUROPEEN

(11) **EP 2 497 774 B1**
(45) Date de publication et mention de la délivrance du brevet: **16.10.2013**
(21) Numéro de dépôt: 12290077.2
(22) Date de dépôt: 07.03.2012
(51) Int. Cl.: C07D 498/04, A61K 31/551

(54) **Dérivés de dihydro-oxazolobenzodiazépinone, procédés de leur préparation et compositions pharmaceutiques contenant ces composés**
Dihydro-oxazolobenzodiazepinon-Derivate, Verfahren zu deren Herstellung und pharmazeutsche Zusammensetzungen, die diese Verbindungen enthalten
Dihydro-oxazolobenzodiazepinone derivatives, processes for their preparation and pharmaceutical compositions comprising these compounds

(30) Priorité: 08.03.2011 FR 1100682
(43) Date de publication de la demande: 12.09.2012
(73) Titulaire: Les Laboratoires Servier, 92284 Suresnes Cedex (FR); EGIS Gyógyszergyár Nyrt, 1106 Budapest (HU)
(72) Inventeur: Ling, István, 1141 Budapest (HU); Barkóczy, József, 1016 Budapest (HU); Antoni, Ferenc, EH10 5RW Edinburgh (GB); Gacsályi, István, 1201 Budapest (HU); Lévay, György, 2092 Budakeszi (HU); Spedding, Michael, 78110 le Vesinet (FR); Hársing, László, H-1025 Budapest (HU)

(56) Documents cités:
- WO-A1-99/07708
- WO-A2-01/04122

## Description

La présente invention concerne de nouveaux dérivés 1,9-dihydro-2H-[1,3]oxazolo [4,5-h][2,3]benzodiazépin-2-one, leur procédé de préparation, et les compositions pharmaceutiques qui les contiennent.

L'acide γ-amino-butyrique (ou GABA) est le principal neurotransmetteur inhibiteur du système nerveux central des mammifères. Dans le prosencéphale, le GABA est principalement synthétisé par les interneurones qui coordonnent les circuits neuronaux complexes *via* les récepteurs GABA_{A} et GABA_{B}. Les récepteurs GABA_{A} sont des canaux chlorures hétéropentamériques ionotropes, comprenant des sous-unités protéiniques α (6 gènes), β (3 gènes), et γ (3 gènes) dans un rapport 2:2:1.

Les benzodiazépines améliorent l'action du GABA sur les récepteurs GABA_{A} en interagissant sur les sites de liaison modulateurs. Les benzodiazépines agonistes non-sélectives provoquent des effets sédatif, hypnotique, anxiolytique, anti-convulsivant, amnésique, anti-nociceptif et myorelaxant.

Les expériences génétiques de knock-in ont montré que la sous-unité α₁ est responsable des effets sédatifs tandis que la sous-unité α₂ et peut-être α₃ sont responsables des effets anxiolytiques des benzodiazépines agonistes. Les ligands des sites de liaisons des benzodiazépines qui produisent les effets contraires *via* la réduction de l'activation du récepteur GABA_{A} induite par le GABA sont appelés « agonistes inverses ». Ces composés montrent une activité bénéfique contre les désordres cognitifs ; cependant, des effets indésirables proconvulsivants et anxiogènes ont empêché la poursuite d'études cliniques plus approfondies de ces composés.

Les fonctions du récepteur GABA_{A} contenant la sous-unité α₅ sont moins bien définies. Chez la souris, la délétion ou la réduction du nombre de récepteurs GABA_{A} contenant la sous-unité α₅ est associée à une amélioration des fonctions cognitives. De plus, le traitement par un agoniste inverse sélectif α₅ produit un effet procognitif amélioré dans plusieurs modèles de rongeurs, tandis que, chez l'homme, il a été observé un effet procognitif sur le déficit de la mémoire induit par l'alcool.

Il existe un important besoin non-satisfait dans le traitement des déficits cognitifs associés à différentes maladies liées à l'âge, à des maladies neurodégénératives ou vasculaires, ou encore à la schizophrénie. Les traitements actuels contre la maladie d'Alzheimer, pathologie ayant la plus forte prévalence, sont basés soit sur l'inhibition de la cholinestérase (e.g. donepezil), soit sur l'antagonisme NMDA (memantine). Cependant, les inhibiteurs de la cholinestérase possèdent un grand nombre d'effets indésirables liés au mécanisme d'action tandis que l'efficacité réelle de la memantine est limitée. De plus, WO 01/04122 et WO 99/07708 divulguent des antagonistes AMPA de type 2,3-benzodiazepine mais aucun produit issu de ces demandes n'a été commercialisé à ce jour. Par conséquent, il est particulièrement intéressant de disposer de nouvelles thérapies ayant une plus grande efficacité et une meilleure tolérabilité.

Les composés de cette invention, outre le fait qu'ils soient nouveaux, possèdent des propriétés particulièrement intéressantes en se fixant sélectivement sur un sous-type réceptoriel du GABA_{A} et en diminuant les effets du GABA.

Plus spécifiquement, la présente invention concerne les composés de formule (I) : 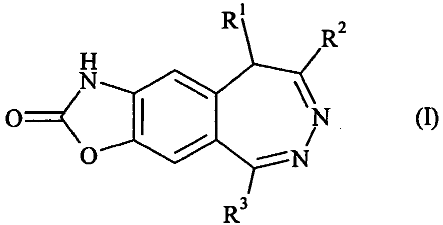 dans laquelle :
➢ R¹ représente un atome d'hydrogène ou un groupement alkyle (C₁-C₄) linéaire ou ramifié ;
➢ R² représente un groupement alkyle (C₁-C₄) linéaire ou ramifié ;
➢ R³ représente un groupement aryle ou hétéroaryle ;
leurs isomères de position, leurs énantiomères, leurs diastéréoisomères, ainsi que leurs sels d'addition à un acide pharmàceutiquement acceptable, leurs solvates, leurs complexes et leurs adduits.

Parmi les acides pharmaceutiquement acceptables, on peut citer à titre non limitatif les acides chlorhydrique, bromhydrique, sulfurique, acétique, trifluoroacétique, lactique, malonique, succinique, glutamique, fumarique, maléique, phosphorique, citrique, oxalique, méthane sulfonique, benzène sulfonique, para-toluènesulfonique, camphorique, etc...

Par groupement aryle, on entend un groupement naphtyle, éventuellement substitué par un ou plusieurs groupements, identiques ou différents, choisis parmi un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylcarbonyle (C₁-C₆) linéaire ou ramifié ; un groupement carboxy ; un groupement alkoxycarbonyle (C₁-C₆) linéaire ou ramifié ; un groupement hydroxy ; un groupement cyano ; un groupement nitro ; un groupement aminocarbonyle non-substitué ou substitué par un ou plusieurs groupements alkyle (C₁-C₆) linéaire ou ramifié ; ou un groupement amino non-substitué ou substitué par un ou deux groupements alkyle (C₁-C₆) linéaire ou ramifié.

Par groupement hétéroaryle, on entend un groupement bicyclique ou tricyclique dans lequel au moins un des cycles est aromatique, comportant de 1 à 3 hétéroatomes, identiques ou différents, choisis parmi l'azote, l'oxygène et le soufre, éventuellement substitué par un ou plusieurs groupements, identiques ou différents, choisis parmi un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylcarbonyle (C₁-C₆) linéaire ou ramifié ; un groupement carboxy ; un groupement alkoxycarbonyle (C₁-C₆) linéaire ou ramifié ; un groupement hydroxy ; un groupement cyano ; un groupement nitro ; un groupement aminocarbonyle non-substitué ou substitué par un ou plusieurs groupements alkyle (C₁-C₆) linéaire ou ramifié ; ou un groupement amino non-substitué ou substitué par un ou deux groupements alkyle (C₁-C₆) linéaire ou ramifié.

Dans les composés de formule (I), R¹ représente préférentiellement un atome d'hydrogène.

De manière avantageuse, les composés de formule (I) sont les composés pour lesquels R² représente un groupement méthyle.

Le groupement R³ représente préférentiellement le groupement hétéroaryle.

Plus particulièrement, les composés de formule (I) préférés sont les composés pour lesquels R³ représente un groupement aromatique bicyclique comportant de 1 à 3 hétéroatomes, identiques ou différents, choisis parmi l'azote, l'oxygène et le soufre, éventuellement substitué par un ou plusieurs groupements, identiques ou différents, choisis parmi un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylcarbonyle (C₁-C₆) linéaire ou ramifié ; carboxy ; un groupement alkoxycarbonyle (C₁-C₆) linéaire ou ramifié ; un groupement hydroxy ; un groupement cyano ; un groupement nitro ; un groupement aminocarbonyle non-substitué ou substitué par un ou plusieurs groupements alkyle (C₁-C₆) linéaire ou ramifié ; ou un groupement amino non-substitué ou substitué par un ou deux groupements alkyle (C₁-C₆) linéaire ou ramifié.

Les composés de formule (I) préférés sont les composés pour lesquels R³ représente un groupement benzothiényl, benzofuranyl ou quinolinyl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

D'autres composés préférés de l'invention sont ceux pour lesquels R³ représente un groupement 1-benzothiényl ou 6-quinolinyl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

Une autre possibilité avantageuse consiste en ce que R³ représente un groupement 1-benzothiényl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

Le groupement R³ représente un groupement 1-benzothién-2-yl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

Les substitutions préférées du groupement hétéroaryle sont l'atome d'halogène tel que le fluor, le chlore, le brome ou l'iode et, plus particulièrement, le fluor ou le chlore ; le groupement trifluorométhyle ; ou le groupement méthyle.

Les composés préférés de l'invention sont le :
- 5-(4-fluoro-1-benzothién-2-yl)-8-méthyl-1,9-dihydro-2*H-*[1,3]oxazolo[4,5-*h*][2,3] benzodiazépin-2-one;
- 8-méthyl-5-(6-quinolinyl)-1,9-dihydro-2*H*-[1,3]oxazolo[4,5-*h*][2,3]benzodiazépin-2-one ;
- 5-(1-benzothién-2-yl)-8-méthyl-1,9-dihydro-2*H*-[1,3]oxazolo[4,5-*h*][2,3] benzodiazépin-2-one ;
- 5-(5-chloro-1-benzothién-2-yl)-8-méthyl-1,9-dihydro-2*H*-[1,3]oxazolo[4,5-*h*] [2,3] benzodiazépin-2-one ;
- 5-[3-chloro-4-(trifluorométhyl)-1-benzothién-2-yl]-8-méthyl-1,9-dihydro-2*H-*[1,3]oxazolo[4,5-*h*][2,3]benzodiazépin-2-one ;
- 8-méthyl-5-[4-(trifluorométhyl)-1-benzothién-2-yl]-1,9-dihydro-2*H*-[1,3]oxazolo [4,5-*h*][2,3]benzodiazépin-2-one ;
- 5-(6-fluoro-1-benzothién-2-yl)-8-méthyl-1,9-dihydro-2*H-*[1,3]oxazolo[4,5-*h*][2,3] benzodiazépin-2-one ;
- 5-(7-fluoro-1-benzothién-2-yl)-8-méthyl-1,9-dihydro-2*H*-[1,3]oxazolo[4,5-*h*][2,3] benzodiazépin-2-one.

Les sels d'addition à un acide pharmaceutiquement acceptable ainsi que les solvates, les complexes et les adduits des composés préférés de l'invention font partie intégrante de l'invention.

L'invention s'étend également au procédé de préparation des composés de formule (I), caractérisé en ce que l'on utilise comme produit de départ un composé de formule (II) : 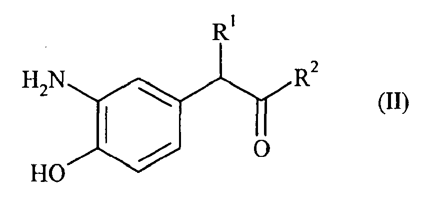 dans laquelle R¹ et R² sont tels que définis dans la formule (I),
composé de formule (II), sous forme libre ou salifiée, qui est ensuite soumis à une réaction de cyclisation en présence de 1,1'-carbonyldiimidazole pour conduire au composé de formule (III) : 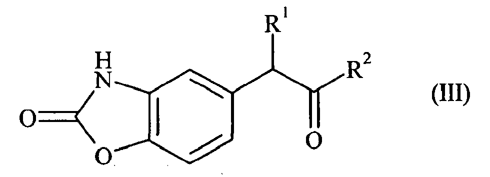 dans laquelle R¹ et R² sont tels que définis dans la formule (I),
qui est mis en réaction avec un agent réducteur pour conduire au composé de formule (IV) : 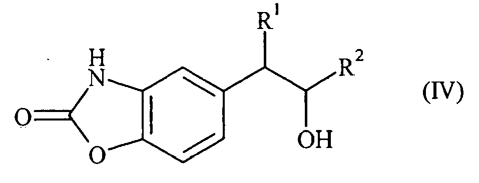 dans laquelle R¹ et R² sont tels que définis dans la formule (I), qui subit ensuite l'action du composé de formule (V) :

R³-CHO (V)

dans laquelle R³ est tel que défini dans la formule (I),
pour conduire au composé de formule (VI) : 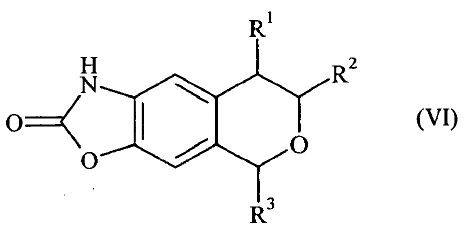 dans laquelle R¹, R² et R³ sont tels que définis dans la formule (I),
qui est ensuite soumis à l'action d'un agent oxydant, suivie de la formation d'un sel, pour conduire au composé de formule (VII) : 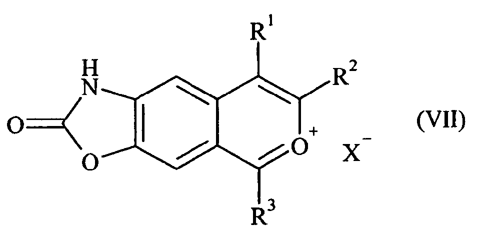 dans laquelle R¹, R² et R³ sont tels que définis dans la formule (I) et X représente un contre-ion tel que ClO₄⁻, Cl⁻, Br⁻, HSO₄⁻,
qui subit ensuite l'action de l'hydrazine pour conduire au composé de formule (I),
composé de formule (I) qui peut être ensuite purifié selon une technique classique de séparation, que l'on transforme, si on le souhaite en ses sels d'addition à un acide pharmaceutiquement acceptable et dont on sépare éventuellement les isomères, s'ils existent, selon une technique classique de séparation.

Une variante avantageuse concerne le procédé de préparation des composés de formule (I) caractérisé en ce que l'on utilise comme produit de départ le composé de formule (III) : 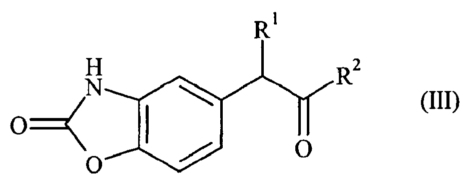 dans laquelle R¹ et R² sont tels que définis dans la formule (I),
qui est soumis à une réaction de bromation pour conduire au composé de formule (VIII) : 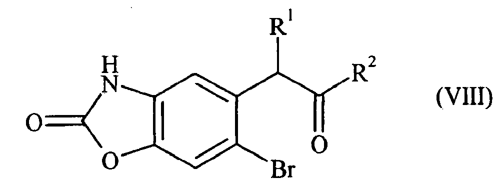 dans laquelle R¹ et R² sont tels que définis précédemment,
qui subit une étape de protection du groupement carbonyle pour conduire au composé de formule (IX) : 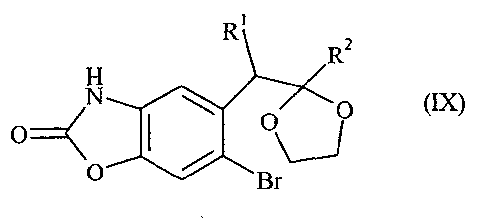 dans laquelle R¹ et R² sont tels que définis précédemment,
qui subit ensuite l'action du composé de formule (X) : 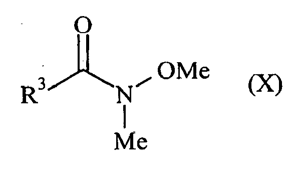 dans laquelle R³ est tel que défini dans la formule (I),
pour conduire au composé de formule (XI) : 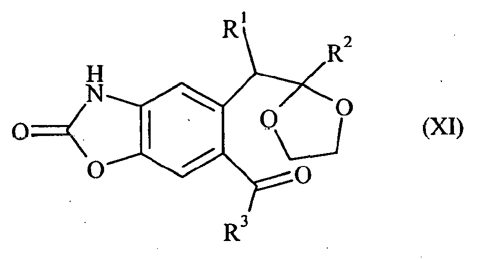 dans laquelle R¹, R² et R³ sont tels que définis dans la formule (I),
qui subit ensuite un réaction de cyclisation pour conduire au composé de formule (VII) : 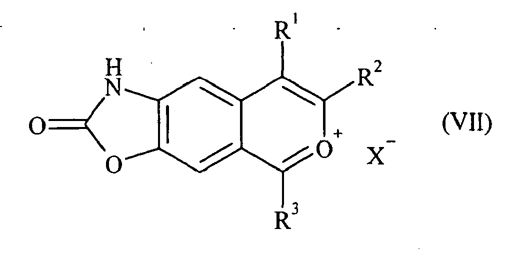 dans laquelle R¹, R² et R³ sont tels que définis dans la formule (I) et X représente un contre-ion tel que ClO₄⁻, Cl⁻, Br⁻, HSO₄⁻,
qui est ensuite soumis à l'action de l'hydrazine pour conduire composé de formule (I),
composé de formule (I) qui peut être ensuite purifié selon une technique classique de séparation, que l'on transforme, si on le souhaite en ses sels d'addition à un acide pharmaceutiquement acceptable et dont on sépare éventuellement les isomères, s'ils existent, selon une technique classique de séparation.

Les composés de formule (II), (V) et (X) sont commerciaux ou aisément accessibles à l'homme du métier par des réactions de chimie classiques ou décrites dans la littérature.

Les composés de la présente invention sont sélectifs de la sous-unité α₅ du récepteur GABA_{A} et diminuent les effets du neurotransmetteur GABA, qui les rendent utiles dans le traitement ou la prévention des maladies psychiatriques et neurologiques caractérisées par des déficits cognitifs, telles que la schizophrénie, la dépression unipolaire, la maladie d'Alzheimer, la démence vasculaire, les maladies du spectre autistique, le syndrome de Down, le syndrome de l'X fragile, la maladie de Parkinson, la maladie d'Huntington. D'autres indications thérapeutiques possibles sont liées à différents états d'anxiété tels que l'anxiété généralisée, la panique avec ou sans agoraphobie, les troubles obsessionnels compulsifs, les troubles de stress post-traumatique et les troubles bipolaires. Les composés de l'invention peuvent être utiles dans les traitement des séquelles d'un accident vasculaire cérébral et des séquelles d'un traumatisme cérébral, rachidien ou médullaire.

Les composés seront utilisés de préférence dans le traitement ou la prévention de la maladie d'Alzheimer, la démence vasculaire telle qu'une démence des suites d'un accident vasculaire cérébral, la maladie d'Huntington et le syndrome de Down.

L'invention s'étend également aux compositions pharmaceutiques renfermant comme principe actif au moins un composé de formule (I) seul ou en combinaison avec un ou plusieurs excipients ou véhicules inertes non toxiques. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, et les suspensions buvables.

La posologie utile varie selon l'âge et le poids du patient, la nature et la sévérité de l'affection ainsi que la voie d'administration. Celle-ci peut être nasale, rectale, parentérale ou orale. D'une manière générale, la posologie unitaire s'échelonne entre 0,1 et 1000 mg pour un traitement en 1 à 3 prises par 24 heures.

Les exemples suivants illustrent l'invention et ne la limitent en aucune façon. Les structures des composés décrits ont été confirmées par les techniques spectroscopiques usuelles (dont la RMN du proton : ls = large singulet ; s = singulet ; d = doublet ; t = triplet ; dd = doublet dédoublé ; m = multiplet).

Les préparations décrites ci-dessous conduisent à des produits de départ utilisés lors de la synthèse des composés de l'invention.

### Préparation 1 : 5-(2-hydroxypropyl)-1,3-benzoxazol-2(3H)-one

### Stade A : 5-(2-oxopropyl)-1,3-benzoxazol-2(3H)-one

A une solution de chlorhydrate de (3-amino-4-hydroxyphényl)acétone (préparé selon EP 101 223 ; Chemistry Letters 1980, 1, 85-88 ; ou J. Org. Chem. 1951, 16, 221-224) (250 mmol) dans le tétrahydrofurane (509 mL), est ajouté le 1,1'-carbonyldiimidazole (48,25 g ; 290 mmol) et le mélange est porté à reflux pendant 2 heures. Après refroidissement à température ambiante, le précipité est filtré et le filtrat est évaporé sous vide. Le résidu est dissous dans l'acétate d'éthyle (400 mL), la solution est lavée avec une solution aqueuse de HCl 5 % (2 x 200 mL) et avec de la saumure (2 x 200 mL), puis la phase organique est concentrée sous vide pour donner le produit du titre sous forme d'un solide.

*Point de fusion: 115-116 °C*

### Stade B : 5-(2-hydroxypropyl)-1,3-benzoxazol-2(3H)-one

A une solution du produit du Stade précédent (180 mmol) dans l'acétate d'éthyle (352 mL) et l'eau (120 mL), est ajouté en plusieurs fois le borohydrure de sodium (2,1 g ; 550 mmol) pendant 20 minutes à 0-10 °C. Le mélange réactionnel est agité à température ambiante jusqu'à ce que la réaction soit complète. Le mélange est ensuite traité par une solution aqueuse de HCl 10 % jusqu'à pH = 2 et, après séparation des phases, la phase aqueuse est extraite avec de l'acétate d'éthyle (3 x 90 mL). Les phases organiques sont rassemblées, séchées sur sulfate de sodium et concentrées sous vide. Le résidu solide est mis en suspension dans l'éther diisopropylique puis filtré pour donner le produit du titre sous forme d'un solide.

*Point de fusion 133-134 °C*

### Préparation 2 : 5-(1-éthyl-2-hydroxypropyl)-1,3-benzoxazol-2(3H)-one

Le produit du titre est obtenu selon le procédé décrit dans la Préparation 1, en utilisant comme réactif de départ le chlorhydrate de 3-(3-amino-4-hydroxyphényl)-2-pentanone à la place du chlorhydrate de 1-(3-amino-4-hydroxyphényl)acétone.

*Point de fusion: 107-109 °C*

### Préparation 3 : 5-[1-(1-hydroxyéthyl)butyl]-1,3-benzoxazol-2(3H)-one

Le produit du titre est obtenu sous forme d'une huile selon le procédé décrit dans la Préparation 1, en utilisant comme réactif de départ le chlorhydrate de 2-amino-4-[1-(1-hydroxyéthyl)butyl]phénol à la place du chlorhydrate de 1-(3-amino-4-hydroxyphényl)acétone.

### Préparation 4 : 5-(2-hydroxybutyl)-1,3-benzoxazol-2(3H)-one

Le produit du titre est obtenu selon le procédé décrit dans la Préparation 1, en utilisant comme réactif de départ le chlorhydrate de 1-(3-amino-4-hydroxyphényl)-2-butanone à la place du chlorhydrate de 1-(3-amino-4-hydroxyphényl)acétone.

*Point de fusion: 117-119 °C*

### EXEMPLE 1: 8-méthyl-5-(2-naphtyl)-1,9-dihydro-2H-[1,3]oxazolo[4,5-h][2,3] benzodiazépin-2-one

### Stade A : 7-méthyl-5-(2-naphtyl)-1,5,7,8-tétrahydro-2H-isochroméno[6,7-d] [1,3]oxazol-2-one

A une suspension du composé de la Préparation 1 (97,0 mmol) et du 2-naphtaldéhyde (94,3 mmol) dans l'acétate d'éthyle (180 mL), est ajoutée une solution anhydre de HCl 15 % dans l'acétate d'éthyle (90 mL). Le mélange réactionnel est agité pendant 20 heures à température ambiante. Une précipitation est observée et le produit attendu sous forme d'une poudre est collecté par filtration.

*Point de fusion: 220-222 °C*

### Stade B : Perchlorate de 7-méthyl-5-(2-naphtyl)-2-oxo-1H,2H-isochroméno[6,7-d] [1,3]oxazol-6-ium

A une solution du produit du Stade précédent (67,5 mmol) dans l'acétone (490 mL), est ajouté goutte à goutte le réactif de Jones (88,63 mL ; 236 mmol) à 0-10 °C pendant 40 minutes. Le mélange est agité à température ambiante jusqu'à ce que la réaction se termine, puis est versé dans de l'eau glacée (2200 mL). Le précipité est filtré, lavé à l'eau (5 x 50 mL), séché puis mis directement en réaction dans l'étape suivante.

A une suspension en produit sec dans l'acétate d'éthyle au reflux (460 mL), est ajouté l'acide perchlorique 70 % (5,87 mL ; 67,5 mmol). Le reflux est maintenu pendant 60 minutes supplémentaires sous forte agitation. Après refroidissement à température ambiante, les cristaux obtenus sont filtrés et séchés pour donner le produit attendu.

*Point de fusion: 304-307 °C*

### Stade C : 8-méthyl-5-(2-naphtyl)-1,9-dihydro-2H-[1,3]oxazolo[4,5-h][2,3] benzodiazépin-2-one

A une solution du produit du Stade précédent (51 mmol) dans le 2-propanol (468 mL), est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié au reflux dans l'acétonitrile.

*Point de fusion: 304-306 °C*

### EXEMPLE 2 : 5-(1-benzofuran-2-yl)-8-méthyl-1,9-dihydro-2H-[1,3]oxazolo[4,5-h] [2,3] benzodiazépin-2-one

### Stade A : 6-bromo-5-(2-oxopropyl)-1,3-benzoxazol-2(3H)-one

A une solution du composé de la Préparation 1 (52 mmol) dans le méthanol (150 mL), est ajouté le *N*-bromosuccinimide (9,8 g ; 55 mmol) en petites portions à 0-10 °C. Le mélange est agité pendant une heure supplémentaire puis évaporé sous vide. Le résidu obtenu est dissous dans l'acétate d'éthyle (250 mL), et la phase organique est lavée avec une solution aqueuse d'hydrogénocarbonate de sodium 5 % (4 x 50 mL), séchée sur MgSO₄ et évaporée sous vide pour donner le produit attendu sous forme d'une solide.

*Point de fusion: 160-162 °C*

### Stade B : 6-bromo-5-[(2-méthyl-1,3-dioxolan-2 yl)méthyl]-1,3-benzoxazol-2(3H)-one

Un mélange du produit du Stade précédent (14,7 g ; 54 mmol), d'éthylène glycol (13,6 mL ; 243 mmol), d'acide *para-*toluène-sulfonique (1 g ; 5 mmol) et de toluène (300 mL) est porté au reflux en utilisant un *Dean-Stark* pendant 6 heures. La solution est refroidie à température ambiante puis versée dans de l'acétate d'éthyle (300 mL). La phase organique est lavée avec une solution aqueuse d'hydrogénocarbonate de sodium 5 % (100 mL), de saumure (100 mL), puis séchée sur MgSO₄ et évaporée sous vide. Le résidu est purifié par chromatographie sur colonne de gel de silice (éluant : dichlorométhane/acétate d'éthyle) pour donner le produit du titre sous forme solide.

*Point de fusion : 117-119 °C*

### Stade C : 6-(1-benzofuran-2-ylcarbonyl)-5-[(2-méthyl-1,3-dioxolan-2-yl)méthyl]-1,3-benzoxazol-2(3H)-one

Une solution de *n*-butyllithium 2.5 M dans l'hexane (10,5 mL ; 2.6 mmol) est ajoutée goutte à goutte à une solution du produit du Stade précédent (3,1 g ; 10 mmol) dans le tétrahydrofurane (120 mL) à - 78 °C. Le mélange réactionnel est ensuite porté à -35 °C, agité pendant 30 minutes, puis le *N*-méthoxy-*N*-méthyl-1-benzofuran-2-carboxamide (16,5 mmol) est ajouté. Le mélange réactionnel est ensuite agité à -35°C pendant 1,5 heures supplémentaires, puis est versé dans une solution saturée en chlorure d'ammonium (150 mL). Après ajout d'acétate d'éthyle (150 mL), la phase organique est lavée à la saumure (100 mL), séchée sur MgSO₄ et évaporée sous vide. Le résidu est purifié par chromatographie sur colonne de gel de silice (éluant : hexane/acétate d'éthyle) pour conduire au produit du titre.

*Point de fusion: 213-215 °C*

### Stade D : Perchlorate de 5-(1-benzofuran-2-yl)-7-méthyl-2-oxo-1H,2H-isochroméno[6,7-d][1,3]oxazol-6-ium

A une suspension du produit du Stade précédent dans l'acétate d'éthyle au reflux (460 mL), est ajouté l'acide perchlorique 70 % (5,87 mL ; 67,5 mmol). Le reflux est maintenu pendant 60 minutes supplémentaires sous forte agitation. Après refroidissement à température ambiante, les cristaux obtenus sont filtrés et séchés pour donner le produit attendu.

*Point de fusion: 318-320 °C*

### Step E: 5-(1-benzofuran-2-yl)-8-méthyl-1, 9-dihydro-2H-[1,3]oxazolo[4,5-h] [2,3]benzodiazépin-2-one

A une solution du produit du Stade précédent (51 mmol) dans le 2-propanol (468 mL), est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié au reflux dans l'acétonitrile.

*Point de fusion : 290-292 °C*

### EXEMPLE 3 : 5-(5-chloro-3-méthyl-1-benzofuran-2-yl)-8-méthyl-1,9-dihydro-2H-[1,3]oxazolo[4,5-h] [2,3]benzodiazépin-2-one

Le produit du titre est obtenu selon le procédé décrit dans l'Exemple 2 en utilisant le 5-chloro-*N*-méthoxy-*N*,3-diméthyl-1-benzofuran-2-carboxamide à la place du N-méthoxy-*N*-méthyl-1-benzofuran-2-carboxamide.

*Point de fusion: 297-298 °C*

### EXEMPLE 4 : 8-méthyl-5-(2-quinolinyl)-1,9-dihydro-2H-[1,3]oxazolo[4,5-h][2,3] benzodiazépin-2-one

A une solution de perchlorate de 7-méthyl-2-oxo-5-(2-quinolinyl)-1*H*,2*H-*isochroméno[6,7-d][1,3]oxazol-6-ium (obtenu selon les Stades A à D décrits dans l'Exemple 2 en utilisant le *N*-méthoxy-*N*-méthyl-2-quinolinecarboxamide à la place du *N*-méthoxy-*N*-méthyl-1-benzofuran-2-carboxamide) (51 mmol) dans le 2-propanol (468 mL), est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié par chromatographie sur colonne de gel de silice (éluant : dichlorométhane/méthanol) pour donner le produit du titre.

*Point de fusion: 304-306 °C*

### EXEMPLE 5 : 8-méthyl-5-(6-quinolinyl)-1,9-dihydro-2H-[1,3]oxazolo[4,5-h][2,3] benzodiazépin-2-one

A une suspension de perchlorate de 7-méthyl-2-oxo-5-(6-quinolinyl)-1*H*,2*H-*isochroméno[6,7-*d*][1,3]oxazol-6-ium (obtenu selon les Stades A à D décrits dans l'Exemple 2 en utilisant le *N*-méthoxy-*N*-méthyl-6-quinolinecarboxamide à la place du *N*-méthoxy-*N*-méthyl-1-benzofuran-2-carboxamide) (51 mmol) dans le 2-propanol (468 mL) est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié par cristallisation dans une mélange diméthylformamide/méthanol.

*Point de fusion: 302-304 °C*

*Analyse spectroscopique de RMN ¹H (500 MHz, DMSO, δ en ppm) : 12,05 (ls, 1H) ; 8,95 (dd, 1H, J = 4,2 et 1,7 Hz) ; 8,44 (dd, 1H, J=8,3 et 1,7Hz); 8,09(m, 3H); 7,57(dd, 1H, J = 8,3 et 4, 2 Hz) ; 7,29 (s, 1H) ; 7, 24 (s, 1H) ; 3,64 (d, 1H, J =12,3 Hz); 2, 89 (d, 1H, J=12,2 Hz) ; 2,10 (s, 3H).*

### EXEMPLE 6 : 5-(1-benzothién-2-yl)-8-méthyl-1,9-dihydro-2H-[1,3]oxazolo[4,5-h] [2,3]benzodiazépin-2-one

A une solution de 5-(1-benzothién-2-yl)-7-méthyl-1,5,7,8-tétrahydro-2*H*-isochroméno [6,7-*d*][1,3]oxazol-2-one (67,5 mmol), obtenu selon le Stade A décrit dans l'Exemple 1 en utilisant le 1-benzothiophène-2-carbaldéhyde à la place du 2-naphtaldéhyde, dans l'acétone (490 mL), est ajouté goutte à goutte le réactif de Jones (88,63 mL ; 236 mmol) à 0-10 °C pendant 40 minutes. Le mélange est agité à température ambiante jusqu'à ce que la réaction se termine, puis est versé dans de l'eau glacée (2200 mL). Le précipité est filtré, lavé à l'eau (5 x 50 mL), séché puis mis directement en réaction dans l'étape suivante. A une suspension en produit sec dans l'acétate d'éthyle au reflux (460 mL), est ajouté l'acide perchlorique 70 % (5,87 mL ; 67,5 mmol). Le reflux est maintenu pendant 60 minutes supplémentaires sous forte agitation. Après refroidissement à température ambiante, le produit brut est isolé puis mis en suspension dans le 2-propanol (434 mL) et l'hydrazine hydratée (6,17 mL ; 127 mmol) est ensuite ajoutée sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est mis au reflux dans l'acétonitrile pendant 30 minutes. Après isolation et séchage, le produit du titre est obtenu.

*Point de fusion: 338-340 °C*

*Analyse spectroscopique de RMN ¹H (500 MHz, DMSO, δ en ppm) : 12,10 (ls, 1H) ; 7,99 (m, 1H) ; 7,86 (m, 1H) ; 7, 69 (s, 1H) ; 7,53 (s, 1H) ; 7,39 (m, 2H) 7, 28 (s, 1 H) ; 3,61 (d, 1H, J = 12,3 Hz) ; 2, 86 (d, 1H, J = 12,2 Hz) ; 2,08 (s, 3H).*

### EXEMPLE 7 : 5-(3-chloro-4-fluoro-1-benzothién-2-yl)-8-méthyl-1,9-dihydro-2H-[1,3]oxazolo[4,5-h] [2,3]benzodiazépin-2-one

Le produit du titre est obtenu selon le procédé décrit dans l'Exemple 1 en utilisant le 3-chloro-4-fluoro-1-benzothiophène-2-carbaldéhyde à la place du 2-naphtaldéhyde.

*Point de fusion : 295-297 °C*

### EXEMPLE 8 : 5-(3-chloro-6-fluoro-1-benzothién-2-yl)-8-méthyl-1,9-dihydro-2H-[1,3]oxazolo[4,5-h] [2,3]benzodiazépin-2-one

A une suspension de perchlorate de 5-(3-chloro-6-fluoro-1-benzothién-2-yl)-7-méthyl-2-oxo-1*H*,2*H-*isochroméno[6,7-*d*][1,3]oxazol-6-ium (obtenu selon les Stades A et B décrits dans l'Exemple 1 en utilisant le 3-chloro-6-fluoro-1-benzothiophène-2-carbaldéhyde à la place du 2-naphtaldéhyde) (51 mmol) dans le 2-propanol (468 mL) est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié par cristallisation dans une mélange diméthylformamide/méthanol.

*Point de fusion: 2 76-2 78 °C*

### EXEMPLE 9 : 5-[3-chloro-4-(trifluorométhyl)-1-benzothién-2-yl]-8-méthyl-1,9-dihydro-2H-[1,3]oxazolo[4,5-h][2,3]benzodiazépin-2-one

Le produit du titre est obtenu selon le procédé décrit dans l'Exemple 1 en utilisant le 3-chloro-4-(trifluorométhyl)-1-benzothiophène-2-carbaldéhyde à la place du 2-naphtaldéhyde.

*Point de fusion: 322-323 °C*

*Analyse spectroscopique de RMN ¹H (500 MHz, DMSO- δ en ppm) : 12,02 (ls, 1H) ; 8,52 (d, 1H, J* = *8,1 Hz) ;* 7,99 *(d, 1H, J* = *7,5 Hz) ;* 7, 72 *(t, 1H, J = 7,9 Hz) ; 7,31 (s, 1H) ; 7,30 (s, 1H) ; 3,77 (d, 1H, J = 12,5 Hz) ; 2,85 (d, 1H, J = 12,3 Hz) ; 2,13 (s, 3H).*

### EXEMPLE 10 : 8-méthyl-5-(3-méthyl-1-benzothién-2-yl)-1,9-dihydro-2H-[1,3]oxazolo [4,5-h] [2,3] benzodiazépin-2-one

Le produit du titre est obtenu selon le procédé décrit dans l'Exemple 1 en utilisant le 3-méthyl-1-benzothiophène-2-carbaldéhyde à la place du 2-naphtaldéhyde.

*Point de fusion: 301-303 °C*

### EXEMPLE 11 : 8-méthyl-5-[3-méthyl-4-(trifluorométhyl)-1-benzothién-2-yl]-1,9-dihydro-2H-[1,3]oxazolo[4,5-h][2,3]benzodiazépin-2-one

Le produit du titre est obtenu selon le procédé décrit dans l'Exemple 1 en utilisant le 3-méthyl-4-(trifluorométhyl)-1-benzothiophène-2-carbaldéhyde à la place du 2-naphtaldéhyde.

*Point de fusion: 313-315 °C*

### EXEMPLE 12 : 8-méthyl-5-[3-méthyl-5-(trifluorométhyl)-1-benzothién-2-yl]-1,9-dihydro-2H-[1,3]oxazolo[4,5-h][2,3]benzodiazépin-2-one

Le produit du titre est obtenu selon le procédé décrit dans l'Exemple 1 en utilisant le 3-méthyl-5-(trifluorométhyl)-1-benzothiophène-2-carbaldéhyde à la place du 2-naphtaldéhyde.

*Point de fusion: 301-303 °C*

### EXEMPLE 13 : 8-méthyl-5-[3-méthyl-6-(trifluorométhyl)-1-benzothién-2-yl]-1,9-dihydro-2H-[1,3]oxazolo[4,5-h][2,3]benzodiazépin-2-one

Le produit du titre est obtenu selon le procédé décrit dans l'Exemple 1 en utilisant le 3-méthyl-6-(trifluorométhyl)-1-benzothiophène-2-carbaldéhyde à la place du 2-naphtaldéhyde.

*Point de fusion: 303-305 °C*

### EXEMPLE 14 : 8-méthyl-5-[3-méthyl-7-(trifluorométhyl)-1-benzothién-2-yl]-1,9-dihydro-2H-[1,3]oxazolo[4,5-h][2,3]benzodiazépin-2-one

Le produit du titre est obtenu selon le procédé décrit dans l'Exemple 1 en utilisant le 3-méthyl-7-(trifluorométhyl)-1-benzothiophène-2-carbaldéhyde à la place du 2-naphtaldéhyde.

*Point de fusion: 292-294 °C*

### EXEMPLE 15 : 5-(3-éthyl-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 1 en utilisant le 3-éthyl-1-benzothiophène-2-carbaldéhyde à la place du 2-naphtaldéhyde.

*Point de fusion: 293-295 °C*

### EXEMPLE 16 : 5-(3-éthyl-4-fluoro-1-benzothién-2-yl)-8-méthyl-1,9-dihydro-2H-[1,3] oxazolo[4,5-h][2,3]benzodiazépin-2-one

A une suspension de perchlorate de 5-(3-éthyl-4-fluoro-1-benzothién-2-yl)-7-méthyl-2-oxo-1*H*,2*H*-isochroméno[6,7-*d*][1,3]oxazol-6-ium (obtenu selon les Stades A et B décrits dans l'Exemple 1 en utilisant le 3-éthyl-4-fluoro-1-benzothiophène-2-carbaldéhyde à la place du 2-naphtaldéhyde) (51 mmol) dans le 2-propanol (468 mL) est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié par chromatographie sur colonne de gel de silice (éluant : dichlorométhane/acétonitrile) pour donner le produit du titre.

*Point de fusion: 29 7-299 °C*

### EXEMPLE 17 : 5-(3-éthyl-4,7-difluoro-1-benzothién-2-yl)-8-méthyl-1,9-dihydro-2H-[1,3]oxazolo[4,5-h] [2,3]benzodiazépin-2-one

Le produit du titre est obtenu selon le procédé décrit dans l'Exemple 8 en utilisant le 3-éthyl-4,7-difluoro-1-benzothiophène-2-carbaldéhyde à la place du 3-chloro-6-fluoro-1-benzothiophène-2-carbaldéhyde.

*Point de fusion: 270-272 °C*

### EXEMPLE 18 : 8-méthyl-5-(3-propyl-1-benzothién-2-yl)-1,9-dihydro-2H-[1,3]oxazolo [4,5-h] [2,3]benzodiazépin-2-one

A une suspension de perchlorate de 5-(3-propyl-1-benzothién-2-yl)-7-méthyl-2-oxo-1*H,*2*H-*isochroméno[6,7-*d*][1,3]oxazol-6-ium (obtenu selon les Stades A et B décrits dans l'Exemple 1 en utilisant le 3-propyl-1-benzothiophène-2-carbaldéhyde à la place du 2-naphtaldéhyde) (51 mmol) dans le 2-propanol (468 mL) est ajoutée l'hydrazine hydratée (6,68 mL ; 133 mmol) sous forte agitation à température ambiante. Le mélange réactionnel est agité pendant 20 heures à température ambiante. Les cristaux obtenus sont ensuite filtrés puis mis sous agitation dans l'eau chaude (1900 mL) pendant 30 minutes. Après filtration et séchage, le solide est purifié par chromatographie sur colonne de gel de silice (éluant : dichlorométhane/acétate d'éthyle) pour donner le produit du titre.

*Point de fusion: 307-308 °C*

### EXEMPLE 19 : 5-(3-butyl-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 18 en utilisant le 3-butyl-1-benzothiophène-2-carbaldéhyde à la place du 3-propyl-1-benzothiophène-2-carbaldéhyde.

*Point de fusion: 278-280 °C*

### EXEMPLE 20 : 8-méthyl-5-[3-(trifluorométhyl)-1-benzothién-2-yl]-1,9-dihydro-2H-[1,3]oxazolo[4,5-h] [2,3]benzodiazépin-2-one

Le produit du titre est obtenu selon le procédé décrit dans l'Exemple 1 en utilisant le 3-(trifluorométhyl)-1-benzothiophène-2-carbaldéhyde à la place du 2-naphtaldéhyde.

*Point de fusion: 260-262 °C*

### EXEMPLE 21 : 5-(4-fluoro-1-benzothién-2-yl)-8-méthyl-1,9-dihydro-2H-[1,3]oxazolo [4,5-h] [2,3]benzodiazépin-2-one

### Stade A : Perchlorate de 5-(4-fluoro-1-benzothién-2-yl)-7-méthyl-2-oxo-1H,2H-isochrornéno[6,7-d][1,3]oxazol-6-ium

Le produit attendu est obtenu selon le procédé décrit dans les Stades A et B de l'Exemple 1 en utilisant le 4-fluoro-1-benzothiophène-2-carbaldéhyde à la place du 2-naphtaldéhyde, ou selon le procédé décrit dans les Stades A à D de l'Exemple 2 en utilisant le 4-fluoro-N-méthoxy-N-méthyl-1-benzothiophène-2-carboxamide à la place de la N-méthoxy-N-méthyl-1-benzofuran-2-carboxamide.

*Point de fusion: 278-280 °C*

### Stade B : 5-(4-fluoro-1-benzothién-2-yl)-8-méthyl-1,9-dihydro-2H-[1,3]oxazolo [4,5-h][2,3]benzodiazépin-2-one

Le produit attendu est obtenu à partir du composé du Stade précédent selon le procédé décrit dans le Stade C de l'Exemple 1.

*Point de fusion: 394-396 °C*

*Analyse spectroscopique de RMN ¹H (500 MHz, DMSO, δ en ppm) : 12,06 (ls, 1H) ; 7,86 (d, 1H, J = 8,1 Hz) ; 7,74 (s, 1H) ; 7,45 (m, 1H) ; 7,45 (s, 1H) ; 7, 28 (s, 1H) ; 7,21 (dd, 1H, J1= 8,1 Hz, J2 = 10, 4 Hz); 3, 62 (d, 1H, J =12,3 Hz) ; 2,85 (d, 1H, J = 12, 2 Hz) ; 2, 08 (s, 3H).*

### EXEMPLE 22 : 5-(4-fluoro-3-méthyl-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 1 en utilisant le 4-fluoro-3-méthyl-1-benzothiophène-2-carbaldéhyde à la place du 2-naphtaldéhyde.

*Point de fusion : 321-323 °C*

### EXEMPLE 23 : 9-éthyl-5-(4-fluoro-1-benzothién-2-yl)-8-méthyl-1,9-dihydro-2H-[1,3] oxazolo[4,5-h][2,3]benzodiazépin-2-one

Le produit du titre est obtenu selon le procédé décrit dans l'Exemple 1 en utilisant le 4-fluoro-1-benzothiophène-2-carbaldéhyde à la place du 2-naphtaldéhyde et le composé de la Préparation 2 à la place du composé de la Préparation 1.

*Point de fusion: 291-292 °C*

### EXEMPLE 24 : 5-(4-fluoro-1-benzothién-2-yl)-8-méthyl-9-propyl-1,9-dihydro-2H-[1,3]oxazolo[4,5-h] [2,3]benzodiazépin-2-one

Le produit du titre est obtenu selon le procédé décrit dans les Stades A et B de l'Exemple 1 en utilisant le 4-fluoro-1-benzothiophène-2-carbaldéhyde à la place du 2-naphtaldéhyde et le composé de la Préparation 3 à la place du composé de la Préparation 1, suivi du procédé décrit dans l'Exemple 18.

*Point de fusion: 299-300 °C*

### EXEMPLE 25 : 5-(4-fluoro-3-méthyl-1-benzothién-2-yl)-8-méthyl-9-propyl-1,9-dihydro-2H-[1,3]oxazolo[4,5-h][2,3]benzodiazépin-2-one

Le produit du titre est obtenu selon le procédé décrit dans l'Exemple 1 en utilisant le 4-fluoro-3-méthyl-1-benzothiophène-2-carbaldéhyde à la place du 2-naphtaldéhyde et le composé de la Préparation 3 à la place du composé de la Préparation 1.

*Point de fusion: 269-271 °C*

### EXEMPLE 26 : 5-(4-fluoro-7-iodo-1-benzothién-2-yl)-8-méthyl-1,9-dihydro-2H-[1,3] oxazolo[4,5-h] [2,3]benzodiazépin-2-one

Le produit du titre est obtenu selon le procédé décrit dans l'Exemple 1 en utilisant le 4-fluoro-7-iodo-1-benzothiophène-2-carbaldéhyde à la place du 2-naphtaldéhyde.

*Point de fusion: 324-326 °C*

### EXEMPLE 27 : 5-(4-chloro-1-benzothién-2-yl)-8-méthyl-1,9-dihydro-2H-[1,3]oxazolo [4,5-h] [2,3] benzodiazépin-2-one

Le produit du titre est obtenu selon le procédé décrit dans l'Exemple 6 en utilisant le 4-chloro-1-benzothiophène-2-carbaldéhyde à la place du 1-benzothiophène-2-carbaldéhyde.

*Point de fusion*: *333-335 °C*

### EXEMPLE 28 : 5-(4-chloro-3-méthyl-1-benzothién-2-yl)-8-méthyl-1,9-dihydro-2H-[1,3] oxazolo[4,5-h] [2,3] benzodiazépin-2-one

Le produit du titre est obtenu selon le procédé décrit dans l'Exemple 1 en utilisant le 4-chloro-3-méthyl-1-benzothiophène-2-carbaldéhyde à la place du 2-naphtaldéhyde.

*Point de fusion: 305-307 °C*

### EXEMPLE 29 : 5-(4-bromo-1-benzothién-2-yl)-8-méthyl-1,9-dihydro-2H-[1,3]oxazolo [4,5-h] [2,3] benzodiazépin-2-one

Le produit du titre est obtenu selon le procédé décrit dans l'Exemple 1 en utilisant le 4-bromo-1-benzothiophène-2-carbaldéhyde à la place du 2-naphtaldéhyde.

*Point de fusion: 336-338 °C*

### EXEMPLE 30 : 5-(4-iodo-1-benzothién-2-yl)-8-méthyl-1,9-dihydro-2H-[1,3]oxazolo [4,5-h] [2,3] benzodiazépin-2-one

Le produit du titre est obtenu selon le procédé décrit dans l'Exemple 1 en utilisant le 4-iodo-1-benzothiophène-2-carbaldéhyde à la place du 2-naphtaldéhyde.

*Point de fusion : 326-328 °C*

### EXEMPLE 31 : 8-méthyl-5-[4-(trifluorométhyl)-1-benzothién-2-yl]-1,9-dihydro-2H-[1,3]oxazolo[4,5-h] [2,3]benzodiazépin-2-one

Le produit du titre est obtenu selon le procédé décrit dans l'Exemple 1 en utilisant le 4-(trifluorométhyl)-1-benzothiophène-2-carbaldéhyde à la place du 2-naphtaldéhyde.

*Point de_fusion: 343-345 °C*

*Analyse spectroscopique de RMN ¹H (500 MHz, DMSO, δ en ppm) : 12,12 (ls, 1H) ; 8,38* (*d*, *1H*, *J* = *8,1 Hz) ; 7,80 (d, 1H, J* = *7,4 Hz) ; 7,70 (s, 1H) ; 7,61* (*t, 1H, J* = *7,8 Hz) ; 7,45* (*s*, 1*H*) *7,29 (s, 1H) 3,64 (d, 1H, J =12,3 Hz) ; 2,88 (d, 1H, J = 12,3 Hz) ; 2, 09 (s, 3H).*

### EXEMPLE 32 : 5-(5-chloro-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 6 en utilisant le 5-chloro-1-benzothiophène-2-carbaldéhyde à la place du 1-benzothiophène-2-carbaldéhyde.

*Point de fusion: 322-324 °C*

*Analyse spectroscopique de RMN ¹H (500 MHz, DMSO, δ en ppm) : 12,11 (ls, 1H) ; 8,03 (d, 1H, J= 8,7 Hz) ; 7,95 (d, 1H, J= 2,1 Hz) ; 7,68 (s, 1H) ; 7,52 (s, 1H) ; 7,44 (dd, 1H, J1 = 2,1 Hz, J2 = 8,5 Hz) ; 7,38 (s, 1H) ; 3,62 (d, 1H, J = 12,5 Hz) ; 2,86 (d, 1H, J =12,2 Hz) ; 2,08 (s,3H).*

### EXEMPLE 33 :5-(5-chloro-3-méthyl-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 6 en utilisant le 5-chloro-3-méthyl-1-benzothiophène-2-carbaldéhyde à la place du 1-benzothiophène-2-carbaldéhyde.

*Point de fusion: 310-312 °C*

### EXEMPLE 34 : 5-(5-fluoro-3-méthyl-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 6 en utilisant le 5-fluoro-3-méthyl-1-benzothiophène-2-carbaldéhyde à la place du 1-benzothiophène-2-carbaldéhyde.

*Point de fusion: 291-292 °C*

### EXEMPLE 35 : 8-méthyl-5-[5-(trifluorométhyl)-1-benzothién-2-yl]-1,9-dihydro-2H-[1,3]oxazolo[4,5-h][2,3]benzodiazépin-2-one

Le produit du titre est obtenu selon le procédé décrit dans l'Exemple 1 en utilisant le 5-(trifluorométhyl)-1-benzothiophène-2-carbaldéhyde à la place du 2-naphtaldéhyde.

*Point de fusion : 282-284 °C*

### EXEMPLE 36 : 5-(6-fluoro-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 6 en utilisant le 6-fluoro-1-benzothiophène-2-carbaldéhyde à la place du 1-benzothiophène-2-carbaldéhyde.

*Point de fusion: 345-347 °C*

### EXEMPLE 37 : 5-(6-fluoro-3-méthyl-1-benzothién-2-yl)-8-méthyl-1,9-dihydro-2H-[1,3]oxazolo[4,5-h] [2,3]benzodiazépin-2-one

Le produit du titre est obtenu selon le procédé décrit dans l'Exemple 1 en utilisant le 6-fluoro-3-méthyl-1-benzothiophène-2-carbaldéhyde à la place du 2-naphtaldéhyde.

*Point de fusion: 286-288 °C*

### EXEMPLE 38 : 8-méthyl-5-[6-(trifluorométhyl)-1-benzothién-2-yl]-1,9-dibydro-2H-[1,3]oxazolo[4,5-h][2,3]benzodiazépin-2-one

Le produit du titre est obtenu selon le procédé décrit dans l'Exemple 6 en utilisant le 6-(trifluorométhyl)-1-benzothiophène-2-carbaldéhyde à la place du 1-benzothiophène-2-carbaldéhyde.

*Point de fusion : 329-331 °C*

### EXEMPLE 39 : 2-(8-méthyl-2-oxo-1,9-dihydro-2H-[1,3]oxazolo[4,5-h][2,3] benzodiazépin-5-yl)-1-benzothiophène-6-carbonitrile

Le produit du titre est obtenu selon le procédé décrit dans l'Exemple 6 en utilisant le 2-formyl-1-benzofuran-6-carbonitrile à la place du 1-benzothiophène-2-carbaldéhyde.

*Point de fusion: 388-390 °C*

### EXEMPLE 40 : 5-(7-fluoro-1-benzothién-2-yl)-8-méthyl-1,9-dihydro-2H-[1,3]oxazolo [4,5-h][2,3]benzodiazépin-2-one

Le produit du titre est obtenu selon le procédé décrit dans l'Exemple 1 en utilisant le 7-fluoro-1-benzothiophène-2-carbaldéhyde à la place du 2-naphtaldéhyde.

*Point de fusion : 324-326 °C*

*Analyse spectroscopique de RMN ¹H (500 MHz-DMSO,_δ en ppm) : 12,09 (bs, 1H) ; 7,73 (m, 1H); 7, 70 (s, 1H) ; 7,63 (d, 1H, J = 3, 7 Hz) ; 7,42 (m, 1H) ; 7,29 (m, 1H) ; 7, 2 7 (s, 1H) ; 3,62 (d, 1H, J= 12,5 Hz) ; 2,86 (d, 1H, J= 12,3 Hz) ; 2,08 (s, 3H).*

### EXEMPLE 41 : 5-(7-fluoro-3-méthyl-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 1 en utilisant le 7-fluoro-3-méthyl-1-benzothiophène-2-carbaldéhyde à la place du 2-naphtaldéhyde.

*Point de fusion : 307-309 °C*

### EXEMPLE 42 : 5-(7-chloro-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 1 en utilisant le 7-chloro-1-benzothiophène-2-carbaldéhyde à la place du 2-naphtaldéhyde.

*Point de fusion : 333-335 °C*

### EXEMPLE 43 : 5-(7-chloro-4-fluoro-1-benzothién-2-yl)-8-méthyl-1,9-dihydro-2H-[1,3]oxazolo[4,5-h] [2,3]benzodiazépin-2-one

Le produit du titre est obtenu selon le procédé décrit dans l'Exemple 1 en utilisant le 7-chloro-4-fluoro-1-benzothiophène-2-carbaldéhyde à la place du 2-naphtaldéhyde.

*Point de fusion : 364-366 °C*

### EXEMPLE 44 : 5-(7-chloro-4-fluoro-3-méthyl-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 8 en utilisant le 7-chloro-4-fluoro-3-méthyl-1-benzothiophène-2-carbaldéhyde à la place du 3-chloro-6-fluoro-1-benzothiophène-2-carbaldéhyde.

*Point de fusion : 319-320 °C*

### EXEMPLE 45 : 5-[7-chloro-4-(trifluorométhyl)-1-benzothién-2-yl]-8-méthyl-1,9-dihydro-2H-[1,3]oxazolo[4,5-h][2,3]benzodiazépin-2-one

Le produit du titre est obtenu selon le procédé décrit dans l'Exemple 1 en utilisant le 7-chloro-4-(trifluorométhyl)-1-benzothiophène-2-carbaldéhyde à la place du 2-naphtaldéhyde.

*Point de fusion : 340-342 °C*

### EXEMPLE 46 : 5-(7-bromo-4-fluoro-1-benzothién-2-yl)-8-méthyl-1,9-dihydro-2H-[1,3]oxazolo[4,5-h][2,3]benzodiazépin-2-one

Le produit du titre est obtenu selon le procédé décrit dans l'Exemple 1 en utilisant le 7-bromo-4-fluoro-1-benzothiophène-2-carbaldéhyde à la place du 2-naphtaldéhyde.

*Point de fusion : 345-347 °C*

### EXEMPLE 47 : 8-méthyl-5-[7-(trifluorométhyl)-1-benzothién-2-yl]-1,9-dihydro-2H-[1,3]oxazolo[4,5-h] [2,3]benzodiazépin-2-one

Le produit du titre est obtenu selon le procédé décrit dans l'Exemple 1 en utilisant le 7-(trifluorométhyl)-1-benzothiophène-2-carbaldéhyde à la place du 2-naphtaldéhyde.

*Point de fusion_: 336-338 °C*

### EXEMPLE 48 : 8-méthyl-5-(1-méthylnaphtho[2,1-b]thien-2-yl)-1,9-dihydro-2H-[1,3] oxazolo[4,5-h] [2,3]benzodiazépin-2-one

Le produit du titre est obtenu selon le procédé décrit dans l'Exemple 8 en utilisant le 1-méthylnaphtho[2,1-*b*]thiophene-2-carbaldéhyde à la place du 3-chloro-6-fluoro-1-benzothiophène-2-carbaldéhyde.

*Point de fusion: 318-320 °C*

### EXEMPLE 49 : 8-éthyl-5-(4-fluoro-1-benzothién-2-yl)-1,5-dihydro-2H-[1,3]oxazolo [4,5-h] [2,3]benzodiazépin-2-one

Le produit du titre est obtenu selon le procédé décrit dans l'Exemple 1 en utilisant le 4-fluoro-1-benzothiophène-2-carbaldéhyde à la place du 2-naphtaldéhyde et le composé de la Préparation 4 à la place du composé de la Préparation 1.

*Point de fusion: 295-297 °C*

### EXEMPLE 50 : 8-éthyl-5-[4-(trifluorométhyl)-1-benzothién-2-yl]-1,5-dihydro-2H-[1,3]oxazolo[4,5-h] [2,3]benzodiazépin-2-one

Le produit du titre est obtenu selon le procédé décrit dans l'Exemple 1 en utilisant le 4-(trifluorométhyl)-1-benzothiophène-2-carbaldéhyde à la place du 2-naphtaldéhyde et le composé de la Préparation 4 à la place du composé de la Préparation 1.

*Point de fusion : 301-303 °C*

### EXEMPLE 51 : 8-éthyl-5-[4-fluoro-3-méthyl-1-benzothién-2-yl]-1,5-dihydro-2H-[1,3]oxazolo[4,5-h] [2,3]benzodiazépin-2-one

Le produit du titre est obtenu selon le procédé décrit dans l'Exemple 1 en utilisant le 4-fluoro-3-méthyl-1-benzothiophène-2-carbaldéhyde à la place du 2-naphtaldéhyde et le composé de la Préparation 4 à la place du composé de la Préparation 1.

*Point de fusion_: 289-291 °C*

### EXEMPLE 52 : 5-(5-fluoro-1-benzothién-2-yl)-8-méthyl-1,9-dihydro-2H-[1,3]oxazolo [4,5-h] [2,3]benzodiazépin-2-one

Le produit du titre est obtenu selon le procédé décrit dans l'Exemple 1 en utilisant le 5-fluoro-1-benzothiophène-2-carbaldéhyde à la place du 2-naphtaldéhyde.

*Point de fusion : 291-293 °C*

### ETUDE PHARMACOLOGIQUE

### EXEMPLE A : Etude de l'activité réceptorielle GABA_{A}

Les composés sont testés sur des cellules HEK-293 (Human Embryonic Kidney) exprimant de manière stable la sous-unité α₅ du récepteur humain GABA_{A} ainsi que les sous-unités beta2 (courte) et gamma2 (longue). Les cellules sont maintenues en présence d'une sélection de trois antibiotiques - néomycine, zéocine et puromycine - dans un milieu de Dulbecco (DMEM) contenant 10 % (v/v) de sérum foetal bovin. La veille de l'expérience, les cellules sont placées dans des plaques 96 puits (densité de 50000 cellules/puits). Ensuite, les cellules sont préincubées pendant 40 minutes avec les composés à tester et traitées avec du GABA. Le potentiel membranaire est contrôlé grâce à un marqueur FMP bleu (Molecular Devices) en suivant les instructions du fabriquant. Les réponses sont enregistrées pendant 120 secondes sur un lecteur de plaques FlexStation3 (Molecular Devices, USA). Les valeurs d'IC₅₀ des composés testés sont déterminées par ajustement de courbe obtenu par régression non-linéaire en utilisant le logiciel SoftMax Pro (Molecular Devices, USA).

| | **alpha5 IC₅₀ [nM]** |
|---|---|
| **Exemple 5** | 430 |
| **Exemple 6** | 130 |
| **Exemple 9** | 250 |
| **Exemple 21** | 200 |
| **Exemple 31** | 500 |
| **Exemple 32** | 172 |
| **Exemple 36** | 100 |
| **Exemple 40** | 227 |

Par conséquent, les composés de l'invention démontrent une excellente affinité et sont sélectifs pour le récepteur alpha5.

### EXEMPLE B: Test de reconnaissance de nouveaux objets chez la souris

Ce test mesure la mémoire de travail non-spatiale chez le rongeur. Il est basé sur la tendance naturelle de l'animal à passer plus de temps à explorer un nouvel objet plutôt qu'un objet familier. La volonté d'explorer un nouvel objet démontre une utilisation de la mémoire d'apprentissage et de reconnaissance.

Au jour 0 (phase de familiarisation), des souris males NMRI sont placées pendant 2,5 minutes dans une boite noire en PVC (32x45x27 cm) sans objet. Le premier jour, les souris sont libres d'explorer deux objets identiques pendant 3 minutes (phase d'acquisition). Le deuxième jour, un des objets est remplacé par un nouveau et la durée d'exploration est chronométrée pour chaque objet sur une période de 4 minutes (phase de rétention). Le pré-traitement par les composés de l'invention est réalisée par voie i.p. le jour de la phase d'acquisition. La durée d'exploration est mesurée par le logiciel TSE System (TSE System GmbH, Bad Homburg, Allemagne).

### Résultats :

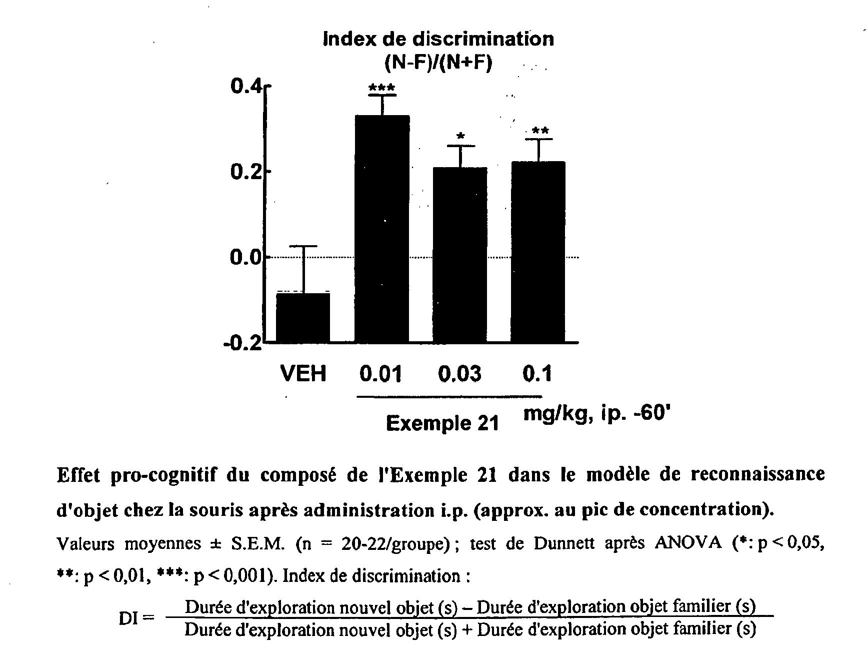

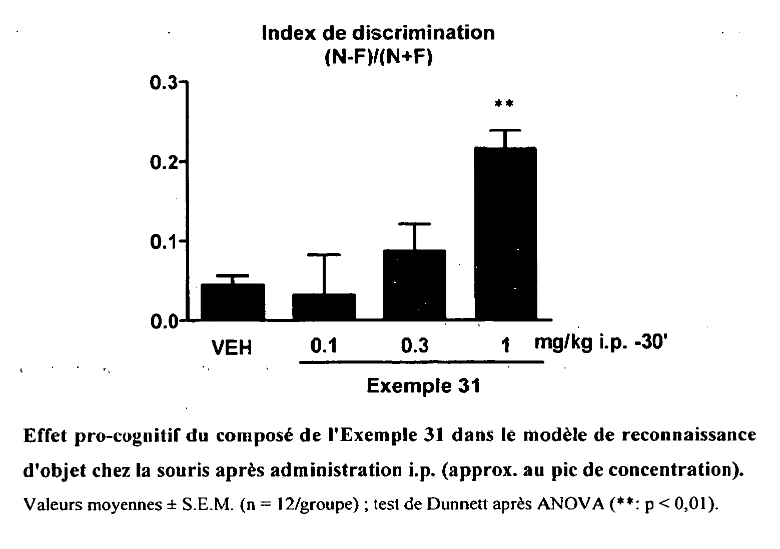

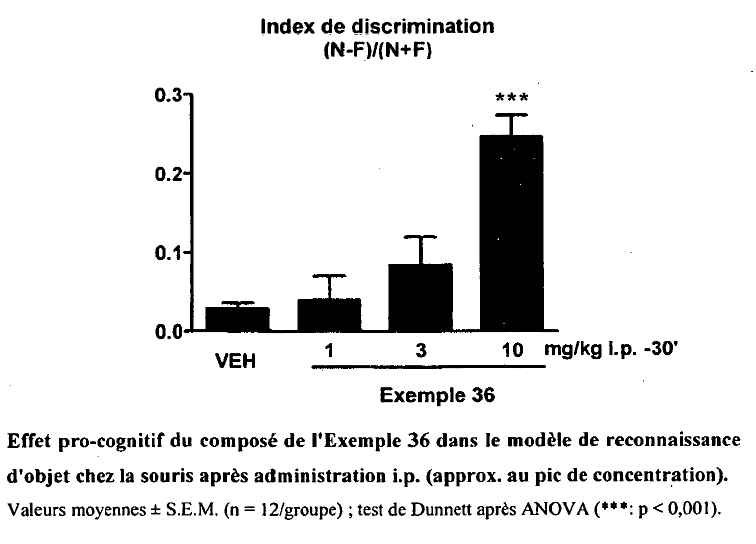

Les résultats indiquent que les composés de l'invention démontrent un effet pro-cognitif dans le modèle de reconnaissance d'objet chez la souris. En particulier, le composé de l'Exemple 21 présente un effet pro-cognitif significatif aux doses de 0,01, 0,03 et 0,1 mg/kg, i.p. De plus, le composé de l'Exemple 31 présente un effet pro-cognitif significatif aux doses de 1 mg/kg, i.p. et le composé de l'Exemple 36 présente un effet procognitif significatif aux doses de 10 mg/kg, i.p.

### EXEMPLE C: Test du labyrinthe radial chez le rat

Ce test est largement utilisé pour évaluer la mémoire de travail et référentielle chez le rongeur. L'appareillage consiste en une petite plate-forme centrale octogonale d'où rayonnent huit corridors différents espacés de manière équidistante. Au bout de chaque corridor, se trouve de la nourriture qui n'est pas visible depuis la plate-forme centrale. Lors de l'expérience, tous les corridors contiennent une récompense et l'animal doit visiter chacun des corridors une seule fois. Chaque visite supplémentaire est considérée comme une erreur.

Le premier jour de l'expérience, les rats sont privés de nourriture pendant 24 heures. L'apprentissage démarre le jour suivant. Les rats sont placés sur la plate-forme centrale du labyrinthe dans lequel les huit corridors sont garnis de nourriture. Les animaux sont libres de manger la nourriture des huit corridors. Si un rat n'a pas consommé la totalité de la nourriture au bout de 20 minutes, il est retiré du labyrinthe.

Les expériences se poursuivent une fois par jour jusqu'à ce que les animaux atteignent l'objectif fixé, celui d'obtenir un nombre total d'erreurs (TE) - c'est-à-dire toute visite supplémentaire d'un corridor - inférieur à 3. Ces animaux seront inclus pour la suite de l'étude. Le dernier jour, les rats sélectionnés sont traités p.o. par la kétamine (10 mg/kg i.p.) co-administrée soit avec un véhicule, soit avec un composé de l'invention. Les tests débutent 120 minutes après administration. Chacun dure au maximum 5 minutes.

### Résultats :

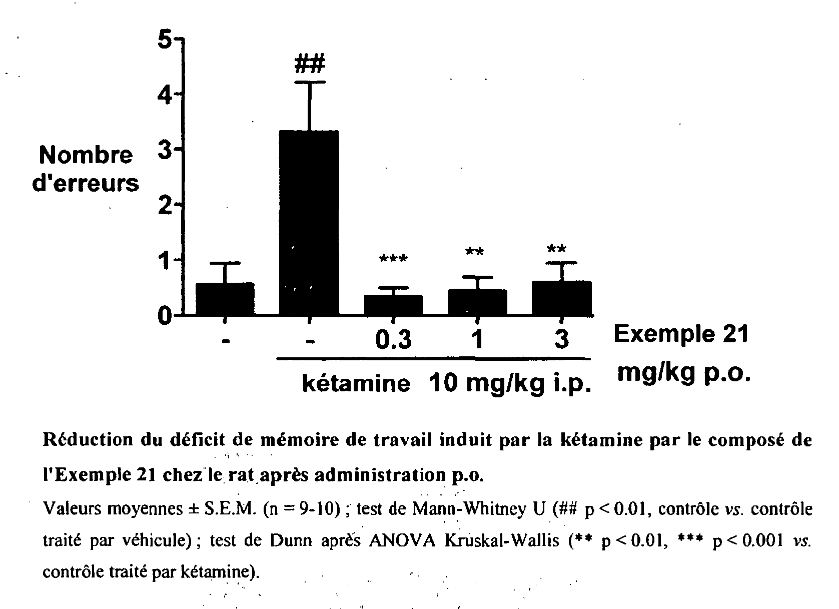

Les résultats indiquent que les composés de l'invention démontrent une amélioration significative de la mémoire des animaux testés. En particulier, le composé de l'Exemple 21 inverse significativement le déficit de mémoire référentielle induit par la kétamine de manière dose-dépendante (de 0,3 à 3 mg/kg p.o.).

### EXEMPLE D: Occlusion de l'artère cérébrale moyenne (ACM) chez la souris

Une ischémie cérébrale focale permanente est produite par électrocoagulation de l'ACM gauche (selon la méthode de Welsh FA et al., J. Neurochem. 1987, 49, 846-851). Des souris NMRI males sont anesthésiées avec le 2,2,2-tribromoéthanol (500 mg/kg i.p., 20 ml/kg). Une incision est pratiquée dans la région temporo-pariétale gauche de la tête entre l'orbite et l'oreille. Le muscle temporal est ensuite incisé puis replié pour laisser apparaître le crâne. Un petit trou de trépan est percé dans la partie latérale extérieure du crâne juste au niveau de l'ACM pour ensuite occlure le tronc de l'ACM par électrocoagulation.

Les composés de l'invention sont administrés par voie i.p. 30 minutes après l'occlusion de l'ACM. Deux jours plus tard, les animaux sont profondément anesthésiés avec du pentobarbital sodique (60 mg/kg i.p., 10 ml/kg) perfusé à travers le coeur avec une solution de chlorure de 2,3,5-triphenyltetrazolium 4 %. Les animaux sont enfin décapités, les cerveaux retirés et placés pendant au moins 24 heures dans une solution saline contenant 8 % de formaline. La mesure de la surface nécrosée est déterminée au moyen d'un système d'analyse d'image (DigiCell pour Windows 4.0).

### Résultats :

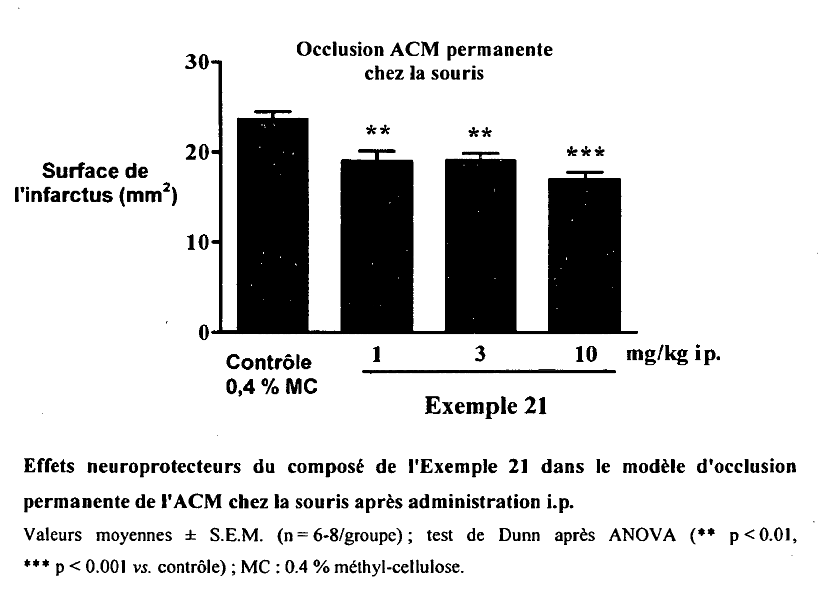

Les résultats indiquent que les composés de l'invention démontrent un effet neuroprotecteur significatif. En particulier, le composé de l'Exemple 21 inhibe significativement les dommages cérébraux pour des doses de 1, 3 et 10 mg/kg i.p. dans le modèle d'occlusion de l'ACM chez la souris.

### EXEMPLE E : Composition pharmaceutique

| | |
|---|---|
| Formule de préparation pour 1000 comprimés dosés à 10 mg en 5-(4-fluoro-1-benzothién-2-yl)-8-méthyl-1,9-dihydro-2*H*-[1,3]oxazolo[4,5-*h*][2,3]benzodiazépin-2-one (Exemple 21) | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I): 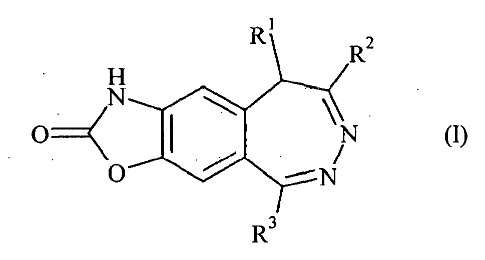 dans laquelle :
➢ R¹ représente un atome d'hydrogène ou un groupement alkyle (C₁-C₄) linéaire ou ramifié ;
➢ R² représente un groupement alkyle (C₁-C₄) linéaire ou ramifié ;
➢ R³ représente un groupement aryle ou hétéroaryle ;
leurs isomères de position, leurs énantiomères, leurs diastéréoisomères, ainsi que leurs sels d'addition à un acide pharmaceutiquement acceptable, leurs solvates, leurs complexes et leurs adduits.

2. Composés de formule (I) selon la revendication 1, **caractérisés en ce que** R¹ représente un atome d'hydrogène.

3. Composés de formule (I) selon la revendication 1, **caractérisés en ce que** R² représente un groupement méthyle.

4. Composés de formule (I) selon la revendication 1, **caractérisés en ce que** R³ représente un groupement hétéroaryle.

5. Composés de formule (I) selon la revendication 1, **caractérisés en ce que** R³ représente un groupement aromatique bicyclique comportant de 1 à 3 hétéroatomes, identiques ou différents, choisis parmi l'azote, l'oxygène et le soufre, éventuellement substitué par un ou plusieurs groupements, identiques ou différents, choisis parmi un atome d'halogène ; un groupement alkyle (C₁-C₆) linéaire ou ramifié non-substitué ou substitué par un ou plusieurs atomes d'halogène ; un groupement alkoxy (C₁-C₆) linéaire ou ramifié ; un groupement alkylcarbonyle (C₁-C₆) linéaire ou ramifié ; un groupement carboxy ; un groupement alkoxycarbonyle (C₁-C₆) linéaire ou ramifié ; un groupement hydroxy ; un groupement cyano ; un groupement nitro ; un groupement aminocarbonyle non-substitué ou substitué par un ou plusieurs groupements alkyle (C₁-C₆) linéaire ou ramifié ; ou un groupement amino non-substitué ou substitué par un ou deux groupements alkyle (C₁-C₆) linéaire ou ramifié.

6. Composés de formule (I) selon la revendication 1, **caractérisés en ce que** R³ représente un groupement benzothiényl ou quinolinyl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

7. Composés de formule (I) selon la revendication 1, **caractérisés en ce que** R³ représente un groupement 1-benzothiényl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

8. Composés de formule (I) selon la revendication 1, **caractérisés en ce que** R³ représente un groupement 1-benzothién-2-yl, éventuellement substitué par un ou plusieurs groupements, identiques ou différents, choisis parmi un atome d'halogène ou un groupement alkyle (C₁-C₄) linéaire ou ramifié non-substitué ou substitué par un ou plusieurs atomes d'halogène.

9. Composés de formule (I) selon la revendication 1, **caractérisés en ce que** R³ représente un groupement 1-benzothién-2-yl, éventuellement substitué par un ou plusieurs groupements, identiques ou différents, choisis parmi un atome de fluor, un atome de chlore, un groupement trifluorométhyle ou un groupement méthyle.

10. Composés de formule (I) selon la revendication 1, qui sont le :
• 5-(4-fluoro-1-benzothién-2-yl)-8-méthyl-1,9-dihydro-2*H*-[1,3]oxazolo[4,5-*h*][2,3] benzodiazépin-2-one ;
• 8-méthyl-5-(6-quinolinyl)-1,9-dihydro-2*H*-[1,3]oxazolo[4,5-*h*][2,3]benzodiazépin-2-one ;
• 5-(1-benzothién-2-yl)-8-méthyl-1,9-dihydro-2*H*-[1,3]oxazolo[4,5-*h*][2,3] benzodiazépin-2-one ;
• 5-(5-chloro-1-benzothién-2-yl)-8-méthyl-1,9-dihydro-2*H*-[1,3]oxazolo[4,5-*h*][2,3] benzodiazépin-2-one ;
• 5-[3-chloro-4-(trifluorométhyl)-1-benzothién-2-yl]-8-méthyl-1,9-dihydro-2*H*-[1,3] oxazolo[4,5-*h*][2,3]benzodiazépin-2-one ;
• 8-méthyl-5-[4-(trifluorométhyl)-1-benzothién-2-yl]-1,9-dihydro-2*H*-[1,3]oxazolo [4,5-*h*][2,3]benzodiazépin-2-one ;
• 5-(6-fluoro-1-benzothién-2-yl)-8-méthyl-1,9-dihydro-2*H*-[1,3]oxazolo[4,5-*h*][2,3] benzodiazépin-2-one ;
• 5-(7-fluoro-1-benzothién-2-yl)-8-méthyl-1,9-dihydro-2*H*-[1,3]oxazolo[4,5-*h*][2,3] benzodiazépin-2-one.

11. Procédé de préparation des composés de formule (I) selon la revendication 1, **caractérisé en ce que** l'on utilise comme produit de départ un composé de formule (II) : 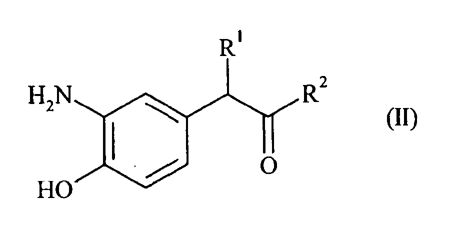 dans laquelle R¹ et R² sont tels que définis dans la formule (I),
composé de formule (II), sous forme libre ou salifiée, qui est ensuite soumis à une réaction de cyclisation en présence de 1,1'-carbonyldiimidazole pour conduire au composé de formule (III) : 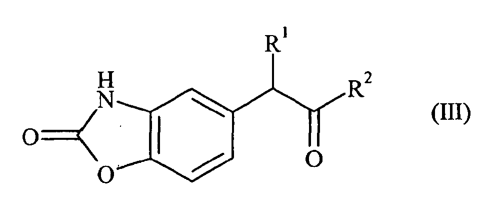 dans laquelle R¹ et R² sont tels que définis dans la formule (I),
qui est mis en réaction avec un agent réducteur pour conduire au composé de formule (IV) : 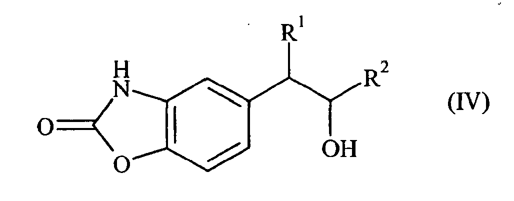 dans laquelle R¹ et R² sont tels que définis dans la formule (I),
qui subit ensuite l'action du composé de formule (V) :
R³-CHO (V)
dans laquelle R³ est tel que défini dans la formule (I),
pour conduire au composé de formule (VI) : 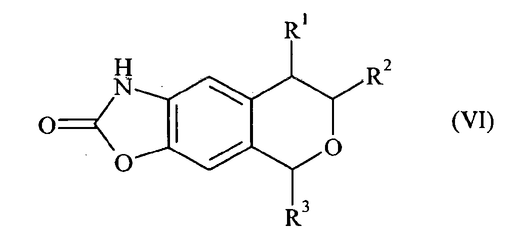 dans laquelle R¹, R² et R³ sont tels que définis dans la formule (I),
qui est ensuite soumis à l'action d'un agent oxydant, suivie de la formation d'un sel, pour conduire au composé de formule (VII) : 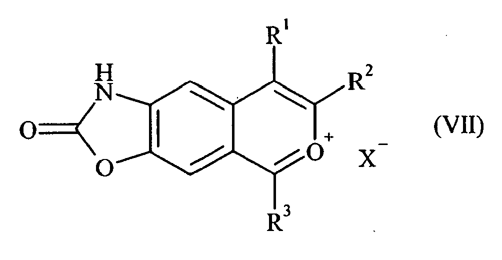 dans laquelle R¹, R² et R³ sont tels que définis dans la formule (I) et X représente un contre-ion tel que ClO₄⁻, Cl⁻, Br⁻, HSO₄⁻,
qui subit ensuite l'action de l'hydrazine pour conduire au composé de formule (I),
composé de formule (I) qui peut être ensuite purifié selon une technique classique de séparation, que l'on transforme, si on le souhaite en ses sels d'addition à un acide pharmaceutiquement acceptable et dont on sépare éventuellement les isomères, s'ils existent, selon une technique classique de séparation.

12. Procédé de préparation des composés de formule (I) selon la revendication 1, **caractérisé en ce que** l'on utilise comme produit de départ un composé de formule (VIII) : 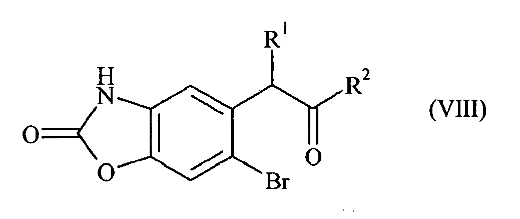 dans laquelle R¹ et R² sont tels que définis précédemment,
qui subit une étape de protection du groupement carbonyle pour conduire au composé de formule (IX) : 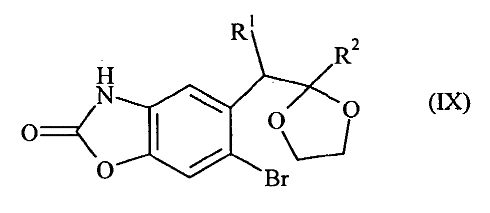 dans laquelle R¹ et R² sont tels que définis précédemment,
qui subit ensuite l'action du composé de formule (X) : 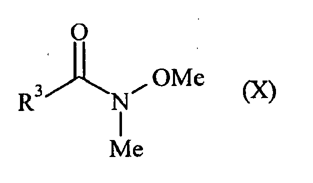 dans laquelle R³ est tel que défini dans la formule (I),
pour conduire au composé de formule (XI) : 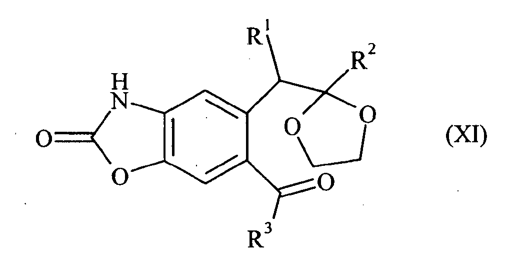 dans laquelle R¹, R² et R³ sont tels que définis dans la formule (I),
qui subit ensuite un réaction de cyclisation pour conduire au composé de formule (VII) : 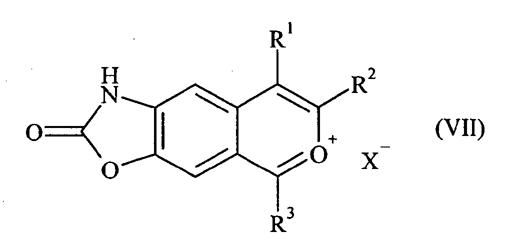 dans laquelle R¹, R² et R³ sont tels que définis dans la formule (I) et X représente un contre-ion tel que ClO₄⁻, Cl⁻, Br⁻, HSO₄⁻,
qui est ensuite soumis à l'action de l'hydrazine pour conduire composé de formule (I),
composé de formule (I) qui peut être ensuite purifié selon une technique classique de séparation, que l'on transforme, si on le souhaite en ses sels d'addition à un acide pharmaceutiquement acceptable et dont on sépare éventuellement les isomères, s'ils existent, selon une technique classique de séparation.

13. Composition pharmaceutique contenant comme principe actif un composé selon l'une quelconque des revendications 1 à 10, en combinaison avec un ou plusieurs véhicules inertes, non toxiques et pharmaceutiquement acceptables.

14. Compositions pharmaceutiques selon la revendication 13 utiles pour le traitement ou la prévention de la schizophrénie, la dépression unipolaire, la maladie d'Alzheimer, la démence vasculaire, les maladies du spectre autistique, le syndrome de Down, le syndrome de l'X fragile, la maladie de Parkinson, la maladie d'Huntington, l'anxiété généralisée, la panique avec ou sans agoraphobie, les troubles obsessionnels compulsifs, les troubles de stress post-traumatique, les troubles bipolaires, les séquelles d'un accident vasculaire cérébrale et les séquelles d'un traumatisme cérébral, rachidien ou médullaire.

15. Utilisation des composés de formule (I) selon l'une quelconque des revendications 1 à 10 pour la fabrication de médicaments utiles pour le traitement ou la prévention de la schizophrénie, la dépression unipolaire, la maladie d'Alzheimer, la démence vasculaire, les maladies du spectre autistique, le syndrome de Down, le syndrome de l'X fragile, la maladie de Parkinson, la maladie d'Huntington, l'anxiété généralisée, la panique avec ou sans agoraphobie, les troubles obsessionnels compulsifs, les troubles de stress post-traumatique, les troubles bipolaires, les séquelles d'un accident vasculaire cérébrale et les séquelles d'un traumatisme cérébral, rachidien ou médullaire.

16. Composés de formule (I) selon l'une quelconque des revendications 1 à 10 utiles pour le traitement ou la prévention de la schizophrénie, la dépression unipolaire, la maladie d'Alzheimer, la démence vasculaire, les maladies du spectre autistique, le syndrome de Down, le syndrome de l'X fragile, la maladie de Parkinson, la maladie d'Huntington, l'anxiété généralisée, la panique avec ou sans agoraphobie, les troubles obsessionnels compulsifs, les troubles de stress post-traumatique, les troubles bipolaires, les séquelles d'un accident vasculaire cérébrale et les séquelles d'un traumatisme cérébral, rachidien ou médullaire.

## Patentansprüche

1. Verbindungen der Formel (I): 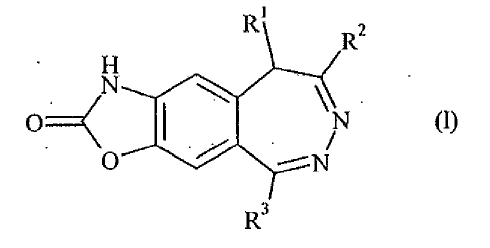 in der:
➢ R¹ ein Wasserstoffatom oder eine geradkettige oder verzweigte (C₁-C₄)-Alkylgruppe bedeutet;
➢ R² eine geradkettige oder verzweigte (C₁-C₄)-Alkylgruppe bedeutet;
➢ R³ eine Aryl- oder Heteroarylgruppe bedeutet;
deren Positionsisomere, deren Enantiomere, deren Diastereoisomere sowie deren Additionssalze mit einer pharmazeutisch annehmbaren Säure, deren Solvate, deren Komplexe und deren Addukte.

2. Verbindungen der Formel (I) nach Anspruch 1, **dadurch gekennzeichnet, dass** R¹ ein Wasserstoffatom bedeutet.

3. Verbindungen der Formel (I) nach Anspruch 1, **dadurch gekennzeichnet, dass** R² eine Methylgruppe bedeutet.

4. Verbindungen der Formel (I) nach Anspruch 1, **dadurch gekennzeichnet, dass** R³ eine Heteroarylgruppe bedeutet.

5. Verbindungen der Formel (I) nach Anspruch 1, **dadurch gekennzeichnet, dass** R³ eine bicyclische aromatische Gruppe, die 1 bis 3 identische oder verschiedene Heteroatome umfasst, ausgewählt aus Stickstoff, Sauerstoff und Schwefel und die gegebenenfalls durch eine oder mehrere identische oder verschiedene Gruppen ausgewählt aus Halogenatomen substituiert ist; eine geradkettige oder verzweigte, nicht-substituierte oder durch ein oder mehrere Halogenatome substituierte (C₁-C₆)-Alkylgruppe; eine geradkettige oder verzweigte (C₁-C₆)-Alkoxygruppe; eine geradkettige oder verzweigte (C₁-C₆)-Alkylcarbonylgruppe, eine Carboxylgruppe; eine geradkettige oder verzweigte (C₁-C₆)-Alkoxycarbonylgruppe; eine Hydroxygruppe; eine Cyanogruppe; eine Nitrogruppe; eine nicht-substituierte oder durch eine oder mehrere geradkettige oder verzweigte (C₁-C₆)-Alkylgruppen substituierte Aminocarbonylgruppe; oder eine nicht-substituierte oder durch eine oder zwei geadkettige oder verzweigte (C₁-C₆)-Alkylgruppen substituierte Aminogruppe bedeutet.

6. Verbindungen der Formel (I) nach Anspruch 1, **dadurch gekennzeichnet, dass** R³ eine Benzothienyl- oder Chinolinylgruppe, die gegebenenfalls durch eine oder mehrere identische oder verschiedene Gruppen ausgewählt aus Halogenatomen substituiert ist, oder eine geradkettige oder verzweigte, nicht-substituierte oder durch ein oder mehrere Halogenatome substituierte (C₁-C₄)-Alkylgruppe bedeutet.

7. Verbindungen der Formel (I) nach Anspruch 1, **dadurch gekennzeichnet, dass** R³ eine 1 -Benzothienylgruppe, die gegebenenfalls durch eine oder mehrere identische oder verschiedene Gruppen ausgewählt aus Halogenatomen substituiert ist, oder eine geradkettige oder verzweigte, nicht-substituierte oder durch ein oder mehrere Halogenatome substituierte (C₁-C₄)-Alkylgruppe bedeutet.

8. Verbindungen der Formel (I) nach Anspruch 1, **dadurch gekennzeichnet, dass** R³ eine 1-Benzothien-2-yl-gruppe, die gegebenenfalls durch eine oder mehrere, identische oder verschiedene Gruppen ausgewählt aus Halogenatomen substituiert ist, oder eine geradkettige oder verzweigte, nicht-substituierte oder durch ein oder mehrere Halogenatome substituierte (C₁-C₄)-Alkylgruppe bedeutet.

9. Verbindungen der Formel (I) nach Anspruch 1, **dadurch gekennzeichnet, dass** R³ eine 1-Benzothien-2-yl-gruppe, die gegebenenfalls durch eine oder mehrere identische oder verschiedene Gruppen ausgewählt aus einem Fluoratom, einem Chloratom substituiert ist, eine Trifluormethylgruppe oder eine Methylgruppe bedeutet.

10. Verbindungen der Formel (I) nach Anspruch 1, nämlich:
• 5-(4-Fluor-1-benzothien-2-yl)-8'-inethyl-1,9-dihydro-2*H*-[1,3]oxazolo[4,5-*h*]-[2,3]benzodiazepin-2-on;
• 8-Methyl-5-(6-chinolinyl)-1,9-dihydro-2*H*-[1,3]oxazolo[4,5-*h*][2,3]benzo-diazepin-2-on;
• 5-(1-Benzothien-2-yl)-8-methyl-1,9-dihydro-2*H*-[1,3]oxazolo[4,5-*h*][2,3]-benzodiazepin-2-on;
• 5-(5-Chlor-1-benzothien-2-yl)-8-methyl-1,9-dihydro-2*H*-[1,3]oxazolo[4,5-*h*]-[2,3]benzodiazepin-2-on;
• 5-[3-Chlor-4-(trifluormethyl)-1-benzothien-2-yl]-8-methyl-1,9-dihydro-2*H-*[1,3]oxazolo[4,5-*h*][2,3]benzodiazepin-2-on;
• 8-Methyl-5-[4-(trifluormethyl)-1-benzothien-2-yl]-1,9-dihydro-2*H*-[1,3]oxa-zolo[4,5-*h*][2,3]benzodiazepin-2-on;
• 5-(6-Fluor-1-benzothien-2-yl)-8-methyl-1,9-dihydro-2*H*-[1,3]oxazolo[4,5-*h*]-[2,3]benzodiazepm-2-on;
• 5-(7-Fluor-1-benzothien-2-yl)-8-methyl-1,9-dihydro-2*H*-[1,3]oxazolo[4,5-*h*]-[2,3]benzodiazepin-2-on.

11. Verfahren zur Herstellung der Verbindungen der Formel (I) nach Anspruch 1, **dadurch gekennzeichnet, dass** man als Ausgangsprodukt eine Verbindung der Formel (II) verwendet: 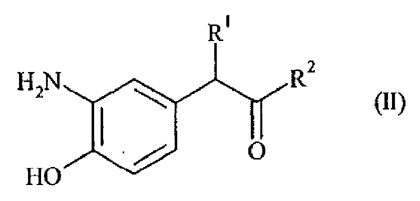 in der R¹ und R² die bezüglich der Formel (I) angegebenen Bedeutungen besitzen, welche Verbindung der Formel (II) in freier Form oder in Salzform anschließend einer Cyclisierungsreaktion in Gegenwart von 1,1'-Carbonyldiimidazol unterworfen wird zur Bildung der Verbindung der Formel (III): 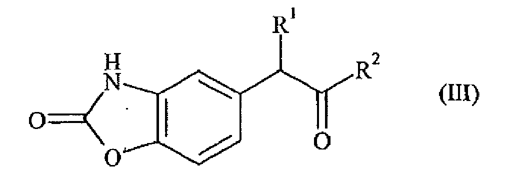 in der R¹ und R² die bezüglich der Formel (I) angegebenen Bedeutungen besitzen, welche mit einem Reduktionsmittel umgesetzt wird zur Bildung der Verbindung der Formel (IV): 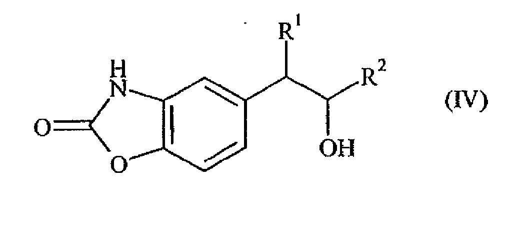 in der R¹ und R² die bezüglich der Formel (I) angegebenen Bedeutungen besitzen, welche anschließend der Einwirkung der Verbindung der Formel (V) unterworfen wird:
R³-CHO (V)
in der R³ die bezüglich der Formel (I) angegebenen Bedeutungen besitzt,
zur Bildung der Verbindung der Formel (VI): 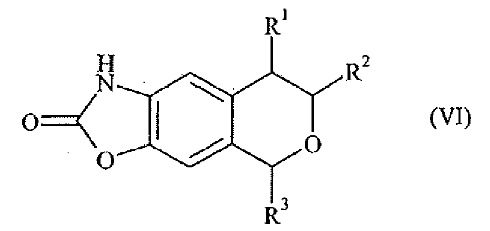 in der R¹, R² und R³ die bezüglich der Formel (I) angegebenen Bedeutungen besitzen, welche anschließend der Einwirkung eines Oxidationsmittels unterworfen wird, gefolgt von der Bildung eines Salzes zur Bildung der Verbindung der Formel (VII): 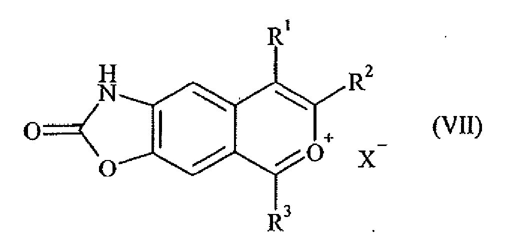 in der R¹, R² und R³ die bezüglich der Formel (I) angegebenen Bedeutungen besitzen und X ein Gegenion wie ClO₄⁻, Cl⁻, Br⁻ oder HSO₄⁻ bedeutet,
welche anschließend der Einwirkung von Hydrazin unterworfen wird zur Bildung der Verbindung der Formel (I),
welche Verbindung der Formel (I) anschließend gemäß einer klassischen Trennmethode gereinigt werden kann, welche man gewünschtenfalls in ihre Additionssalze mit einer pharmazeutisch annehmbaren Säure überführt und welche man gegebenenfalls mit Hilfe einer klassischen Trennmethode in ihre Isomeren, falls sie existieren, auftrennt.

12. Verfahren zur Herstellung der Verbindungen der Formel (I) nach Anspruch 1, **dadurch gekennzeichnet, dass** man als Ausgangsprodukt eine Verbindung der Formel (VIII) verwendet: 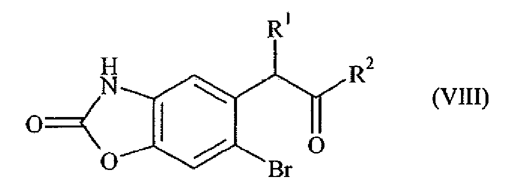 in der R¹ und R² die oben angegebenen Bedeutungen besitzen,
welche man einer Stufe des Schutzes der Carbonylgruppe unterzieht zur Bildung der Verbindung der Formel (IX): 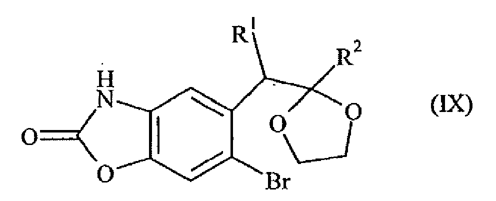 in der R¹ und R² die oben angegebenen Bedeutungen besitzen,
welche anschließend der Einwirkung der Verbindung der Formel (X) unterworfen wird: 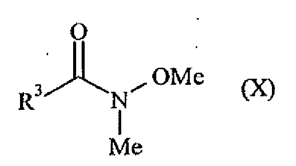 in der R³ die bezüglich der Formel (I) angegebenen Bedeutungen besitzt, zur Bildung der Verbindung der Formel (XI): 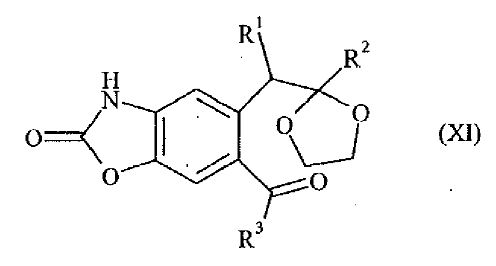 in der R¹, R² und R³ die bezüglich der Formel (I) angegebenen Bedeutungen besitzen, welche man anschließend einer Cyclisierungsreaktion unterwirft zur Bildung der Verbindung der Formel (VII): 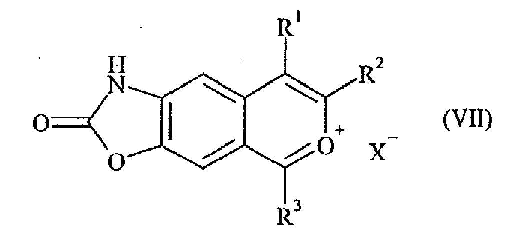 in der R¹, R² und R³ die bezüglich der Formel (I) angegebenen Bedeutungen besitzen und X ein Gegenion wie ClO₄⁻, Cl⁻, Br⁻ oder HSO₄⁻ bedeutet,
welche anschließend der Einwirkung von Hydrazin unterworfen wird zur Bildung der Verbindung der Formel (I),
welche Verbindung der Formel (I) anschließend gemäß einer klassischen Trennmethode gereinigt werden kann, welche man gewünschtenfalls in ihre Additionssalze mit einer pharmazeutisch annehmbaren Säure überführt und welche man gegebenenfalls mit Hilfe einer klassischen Trennmethode in ihre Isomeren, falls sie existieren, auftrennt.

13. Pharmazeutische Zubereitung enthaltend als Wirkstoff eine Verbindung nach einem der Ansprüche 1 bis 10 in Kombination mit einem oder mehreren inerten, nichttoxischen und pharmazeutisch annehmbaren Hilfsstoffen.

14. Pharmazeutische Zubereitungen nach Anspruch 13, nützlich zur Behandlung oder zur Vorbeutung der Schizophrenie, der unipolaren Depression, der Alzheimerschen Krankheit, der vaskulären Demenz, Erkrankungen des autistischen Spektrums, des Down-Syndroms, des Fragilen-X-Syndroms, der Parkinsonschen Krankheit, der Huntingtonschen Krankheit, von allgemeinen Ängsten, Panik mit oder ohne Agoraphobie, kompulsiven Obsessionsstörungen, posttraumatischen Stressstörungen, bipolaren Störungen, Folgen eines Gehirngefäßvorfalls und Folgen eines Gehirn-, Rückenmarks- oder Medullatraumas.

15. Verwendung der Verbindungen der Formel (I) nach einem der Ansprüche 1 bis 10 für die Herstellung von Arzneimitteln, die nützlich sind zur Behandlung oder der Vorbeutung der Schizophrenie, der unipolaren Depression, der Alzheimerschen Krankheit, der vaskulären Demenz, Erkrankungen des autistischen Spektrums, des Down-Syndroms, des Fragilen-X-Syndroms, der Parkinsonschen Krankheit, der Huntingtonschen Krankheit, allgemeinen Ängsten, Panik mit oder ohne Agoraphobie, kompulsiven Obsessionsstörungen, posttraumatischen Stressstörungen, bipolaren Störungen, Folgen eines zerebralen Gefäßvorfalls und Folgen eines Gehirn-, Rückenmarks- oder Medullatraumas.

16. Verbindungen der Formel (I) nach einem der Ansprüche 1 bis 10, nützlich zur Behandlung oder der Vorbeugung der Schizophrenie, der unipolaren Depression, der Alzheimerschen Krankheit, der vaskulären Demenz, Erkrankungen des autistischen Spektrums, des Down-Syndroms, des Fragilen-X-Syndroms, der Parkinsonschen Krankheit, der Huntingtonschen Krankheit, von allgemeinen Ängsten, Panik mit oder ohne Agoraphobie, kompulsiven Obsessionsstörungen, posttraumatischen Stressstörungen, bipolaren Störungen, Folgen eines zerebralen Gefäßvorfalls und Folgen eines Gehirn-, Rückenmarks- oder Medullatraumas.

## Claims

1. Compounds of formula (I): 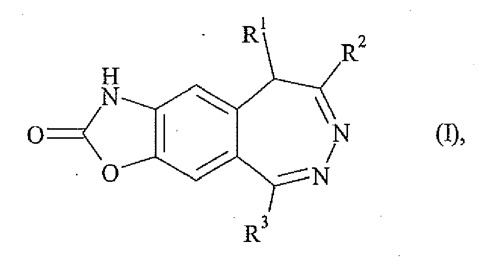 wherein:
➢ R¹ represents a hydrogen atom or a linear or branched (C₁-C₄)alkyl group;
➢ R² represents a linear or branched (C₁-C₄)alkyl group;
➢ R³ represents an aryl or heteroaryl group;
their positional isomers, their enantiomers, their diastereoisomers, and also their addition salts with a pharmaceutically acceptable acid, their solvates, their complexes and their adducts.

2. Compounds of formula (I) according to claim 1, **characterised in that** R¹ represents a hydrogen atom.

3. Compounds of formula (I) according to claim 1, **characterised in that** R² represents a methyl group.

4. Compounds of formula (I) according to claim 1, **characterised in that** R³ represents a heteroaryl group.

5. Compounds of formula (I) according to claim 1, **characterised in that** R³ represents a bicyclic aromatic group containing from 1 to 3 identical or different hetero atoms selected from nitrogen, oxygen and sulphur, optionally substituted by one or more identical or different groups selected from a halogen atom; a linear or branched (C₁-C₆)alkyl group which is unsubstituted or substituted by one or more halogen atoms; a linear or branched (C₁-C₆)alkoxy group; a linear or branched (C₁-C₆)alkylcarbonyl group; a carboxy group; a linear or branched (C₁-C₆)alkoxycarbonyl group; a hydroxy group; a cyano group; a nitro group; an aminocarbonyl group which is unsubstituted or substituted by one or more linear or branched (C₁-C₆)alkyl groups; and an amino group which is unsubstituted or substituted by one or two linear or branched (C₁-C₆)alkyl groups.

6. Compounds of formula (I) according to claim 1, **characterised in that** R³ represents a benzothienyl or quinolyl group, optionally substituted by one or more identical or different groups selected from a halogen atom and a linear or branched (C₁-C₄)alkyl group which is unsubstituted or substituted by one or more halogen atoms.

7. Compounds of formula (I) according to claim 1, **characterised in that** R³ represents a 1-benzothienyl group, optionally substituted by one or more identical or different groups selected from a halogen atom and a linear or branched (C₁-C₄)alkyl group which is unsubstituted or substituted by one or more halogen atoms.

8. Compounds of formula (I) according to claim 1, **characterised in that** R³ represents a 1-benzothien-2-yl group, optionally substituted by one or more identical or different groups selected from a halogen atom and a linear or branched (C₁-C₄)alkyl group which is unsubstituted or substituted by one or more halogen atoms.

9. Compounds of formula (I) according to claim 1, **characterised in that** R³ represents a 1-benzothien-2-yl group, optionally substituted by one or more identical or different groups selected from a fluorine atom, a chlorine atom, a trifluoromethyl group and a methyl group.

10. Compounds of formula (I) according to claim 1, which are:
• 5-(4-fluoro-1-benzothien-2-yl)-8-methyl-1,9-dihydro-2*H*-[1,3]oxazolo[4,5-*h*][2,3]-benzodiazepin-2-one;
• 8-methyl-5-(6-quinolyl)-1,9-dihydro-2*H*-[1,3]oxazolo[4,5-*h*][2,3]benzodiazepin-2-one;
• 5-(1-benzothien-2-yl)-8-methyl-1,9-dihydro-2*H*-[1,3]oxazolo[4,5-*h*][2,3]benzo-diazepin-2-one;
• 5-(5-chloro-1-benzothien-2-yl)-8-methyl-1,9-dihydro-2*H*-[1,3]oxazolo[4,5-*h*][2,3]-benzodiazepin-2-one;
• 5-[3-chloro-4-(trifluoromethyl)-1-benzothien-2-yl]-8-methyl-1,9-dihydro-2*H*-[1,3]-oxazolo[4,5-*h*][2,3]benzodiazepin-2-one;
• 8-methyl-5-[4-(trifluoromethyl)-1-benzothien-2-yl]-1,9-dihydro-2*H*-[1,3]oxazolo-[4,5-*h*][2,3]benzodiazepin-2-one;
• 5-(6-fluoro-1-benzothien-2-yl)-8-methyl-1,9-dihydro-2*H*-[1,3]oxazolo[4,5-*h*][2,3]-benzodiazepin-2-one;
• 5-(7-fluoro-1-benzothien-2-yl)-8-methyl-1,9-dihydro-2*H*-[1,3]oxazolo[4,5-*h*][2,3]-benzodiazepin-2-one.

11. Process for the preparation of compounds of formula (I) according to claim 1, **characterised in that** there is used as starting material a compound of formula (II): 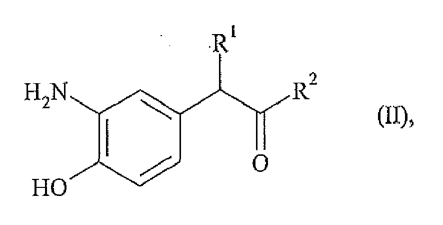 wherein R¹ and R² are as defined for formula (I),
which compound of formula (II), in free form or salt form, is then subjected to a cyclisation reaction in the presence of 1,1'-carbonyldiimidazole to yield the compound of formula (III): 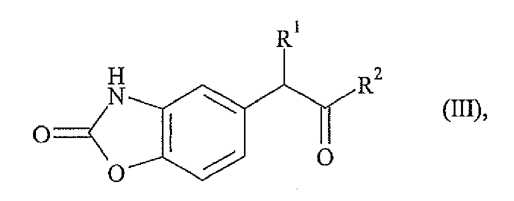 wherein R¹ and R² are as defined for formula (I),
which is reacted with a reducing agent to yield the compound of formula (IV): 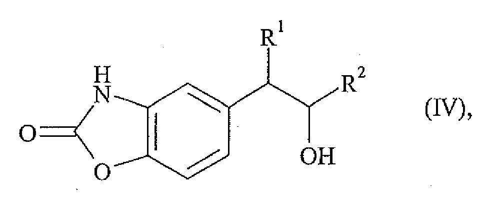 wherein R¹ and R² are as defined for formula (I),
which is then subjected to the action of the compound of formula (V):
R³-CHO (V),
wherein R³ is as defined for formula (I),
to yield the compound of formula (VI): 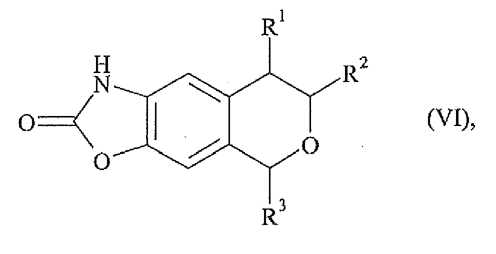 wherein R¹, R² and R³ are as defined for formula (I),
which is then subjected to the action of an oxidising agent, followed by formation of a salt, to yield the compound of formula (VII): 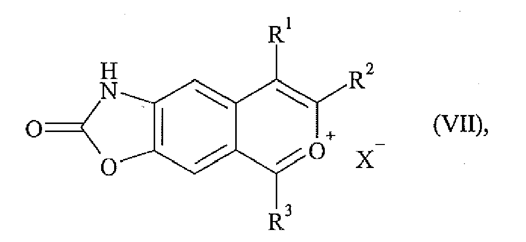 wherein R¹, R² and R³ are as defined for formula (I) and X represents a counter-ion such as ClO₄⁻, Cl⁻, Br⁻, HSO₄⁻,
which is then subjected to the action of hydrazine to yield the compound of formula (I),
which compound of formula (I) may then be purified according to a conventional separation technique, is converted, if desired, into its addition salts with a pharmaceutically acceptable acid and is separated, where appropriate, into its isomers, if they exist, according to a conventional separation technique.

12. Process for the preparation of compounds of formula (I) according to claim 1, **characterised in that** there is used as starting material a compound of formula (VIII): 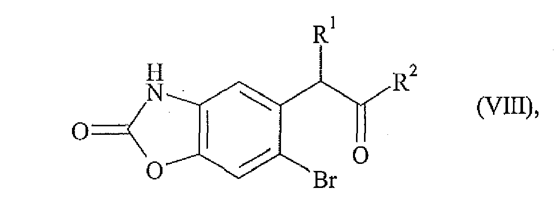 wherein R¹ and R² are as defined for formula (I),
which is subjected to a step of protection of the carbonyl group to yield the compound of formula (IX): 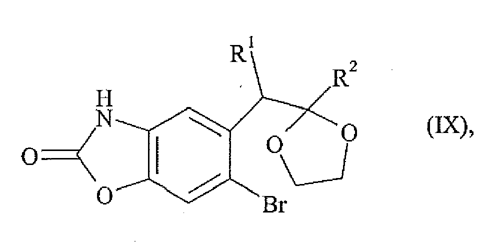 wherein R¹ and R² are as defined hereinbefore,
which is then subjected to the action of the compound of formula (X): 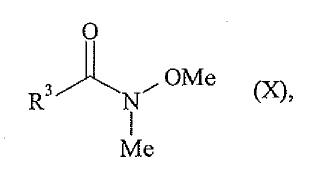 wherein R³ is as defined for formula (I),
to yield the compound of formula (XI): 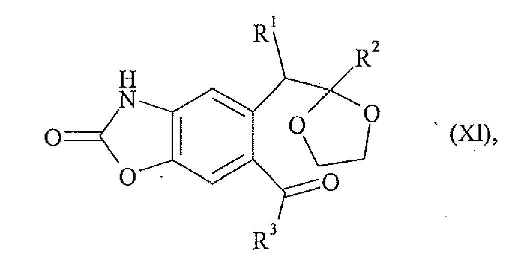 wherein R¹, R² and R³ are as defined for formula (I),
which is then subjected to a cyclisation reaction to yield the compound of formula (VII): 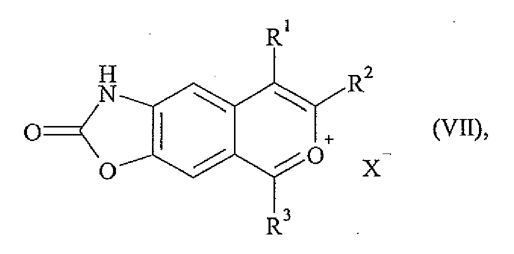 wherein R¹, R² and R³ are as defined for formula (I) and X represents a counter-ion such as ClO₄⁻, Cl⁻, Br⁻, HSO₄⁻,
which is then subjected to the action of hydrazine to yield the compound of formula (I),
which compound of formula (I) may then be purified according to a conventional separation technique, is converted, if desired, into its addition salts with a pharmaceutically acceptable acid and is separated, where appropriate, into its isomers, if they exist, according to a conventional separation technique.

13. Pharmaceutical composition comprising as active ingredient a compound according to any one of claims 1 to 10 in combination with one or more inert, non-toxic, pharmaceutically acceptable carriers.

14. Pharmaceutical compositions according to claim 13 for use in the treatment or prevention of schizophrenia, unipolar depression, Alzheimer's disease, vascular dementia, autism spectrum disorders, Down's syndrome, fragile X syndrome, Parkinson's disease, Huntington's disease, generalised anxiety, panic disorder with or without agoraphobia, obsessive-compulsive disorders, post-traumatic stress disorders, bipolar disorders, sequelae of a cerebral vascular accident and sequelae of brain, spine or medullary trauma.

15. Use of compounds of formula (I) according to any one of claims -1 to 10 in the manufacture of medicaments for use in the treatment or prevention of schizophrenia, unipolar depression, Alzheimer's disease, vascular dementia, autism spectrum disorders, Down's syndrome, fragile X syndrome, Parkinson's disease, Huntington's disease, generalised anxiety, panic disorder with or without agoraphobia, obsessive-compulsive disorders, post-traumatic stress disorders, bipolar disorders, sequelae of a cerebral vascular accident and sequelae of brain, spine or medullary trauma.

16. Compounds of formula (I) according to any one of claims 1 to 10 for use in the treatment or prevention of schizophrenia, unipolar depression, Alzheimer's disease, vascular dementia, autism spectrum disorders, Down's syndrome, fragile X syndrome, Parkinson's disease, Huntington's disease, generalised anxiety, panic disorder with or without agoraphobia, obsessive-compulsive disorders, post-traumatic stress disorders, bipolar disorders, sequelae of a cerebral vascular accident and sequelae of brain, spine or medullary trauma.
